# EUROPEAN PATENT SPECIFICATION

(11) **EP 4 149 447 B1**
(45) Date of publication and mention of the grant of the patent: **30.09.2026**
(21) Application number: 21726608.9
(22) Date of filing: 11.05.2021
(51) Int. Cl.: A61K 31/05, A61K 31/352, A61K 31/427, A61K 31/513, A61K 31/706, A61P 31/14

(54) **CANNABIDIOL FORMULATIONS FOR USE IN THE TREATMENT SARS-COV-2 INFECTION**
CANNABIDIOLFORMULIERUNGEN ZUR VERWENDUNG BEI DER BEHANDLUNG VON SARS-COV-2-INFEKTION
FORMULATIONS DE CANNABIDIOL DESTINÉES À ÊTRE UTILISÉES DANS LE TRAITEMENT D'UNE INFECTION PAR LE SARS-COV-2

(30) Priority: 11.05.2020 WO PCT/EP2020/063086; 16.04.2021 EP 21168856
(43) Date of publication of application: 22.03.2023
(73) Proprietor: ADD Advanced Drug Delivery Technologies, Ltd., 4133 Pratteln (CH)
(72) Inventor: NOWAK, Reinhard, 79589 Binzen (DE); NOWAK, Mirko, 79540 Lörrach (DE); NOWAK, Jesko Jay, 79540 Lörrach (DE); PÖLLINGER, Norbert, 79379 Müllheim (DE)
(74) Representative: Breuer, Markus
(86) International application number: PCT/EP2021/062495
(87) International publication number: WO 2021/228863

(56) References cited:
- WO-A1-2017/072774
- WO-A1-2019/155337
- GB-A- 2 542 797
- BYRAREDDY SIDDAPPA N ET AL: "SARS-CoV2 induced respiratory distress: Can cannabinoids be added to anti-viral therapies to reduce lung inflammation?", BRAIN, BEHAVIOR AND IMMUNITY, ACADEMIC PRESS, SAN DIEGO, CA, US, vol. 87, 28 April 2020 (2020-04-28), pages 120 - 121, XP086197997, ISSN: 0889-1591, [retrieved on 20200428], DOI: 10.1016/J.BBI.2020.04.079
- BO WANG ET AL: "In Search of Preventative Strategies: Novel Anti-Inflammatory High-CBD <em>Cannabis Sativa</em> Extracts Modulate ACE2 Expression in COVID-19 Gateway Tissues", 17 April 2020 (2020-04-17) - 15 July 2021 (2021-07-15), XP055765015, Retrieved from the Internet <URL:https://www.preprints.org/manuscript/202004.0315/v1> DOI: 10.20944/preprints202004.0315.v1
- STEPHEN MAMBER ET AL: "Could Unconventional Immunomodulatory Agents Help Alleviate COVID-19 Symptoms and Severity?", 1 April 2020 (2020-04-01), XP055765043, Retrieved from the Internet <URL:https://www.preprints.org/manuscript/202004.0014/v1> DOI: 10.20944/preprints202004.0014.v1
- CAROL SHOSHKES REISS: "Cannabinoids and Viral Infections", PHARMACEUTICALS, vol. 3, no. 6, 9 June 2010 (2010-06-09), pages 1873 - 1886, XP055765009, DOI: 10.3390/ph3061873
- MEHTA PUJA ET AL: "COVID-19: consider cytokine storm syndromes and immunosuppression", THE LANCET, ELSEVIER, AMSTERDAM, NL, vol. 395, no. 10229, 16 March 2020 (2020-03-16), pages 1033 - 1034, XP086105444, ISSN: 0140-6736, [retrieved on 20200316], DOI: 10.1016/S0140-6736(20)30628-0

## Description

### Field of the Invention

The present invention relates to uses and formulations of cannabinoids, in particular of cannabidiol. According to the invention, the cannabinoids, in particular cannabidiol, are used for the treatment of patients suffering from COVID-19, a disease caused by the coronavirus SARS-Cov-2.

The invention also provides formulations for oral administration of cannabinoids, in particular of cannabidiol. These formulations are useful for treating patients suffering from COVID-19.

### Background of the Invention

Coronavirus disease 2019 (COVID-19), an infectious disease caused by severe acute respiratory syndrome coronavirus 2 (SARS-CoV-2), was first identified in December 2019 in Wuhan, China, and has since spread globally, resulting in the coronavirus pandemic. Due to the highly divergent rate of testing amongst the different populations, mortality of the disease is still uncertain as the number of infected persons is not known. Furthermore, there are methodological concerns regarding affiliations of deaths to the underlying disease. However, currently there is reason to assume that the mortality rate is at least similar as or even higher than the mortality rate of <1 % from influenza. In addition, COVID-19 is more contagious than influenza: the estimated basic reproduction numbers (R0) range between 1.4 and 1.6 for influenza and between 2 and 3 for COVID-19.

Based on interim guidance of the WHO, management of patients with COVID-19 is composed of symptomatic treatment, monitoring, anti-microbial treatment of co-infections and management of disease complications such as acute respiratory distress syndrome (ARDS) and sepsis.

While meanwhile numerous clinical studies have been initiated to test various drugs and treatment regimens, there is still an urgent need for further treatment options.

It has recently been suggested that certain cannabinoids may have utility in the treatment of COVID-19. An *in vitro* cell culture study suggests that, in an artificial model of inflammation, certain *Cannabis sativa* extracts down-regulate ACE2, the receptor for SARS-CoV-2, and also down-regulate serine protease TMPRSS2, another critical protein required for SARS-CoV-2 entry into host cells (B. Wang *et al.* (2020)). In Search of Preventative Strategies: Novel Anti-Inflammatory High-CBD Cannabis Sativa Extracts Modulate ACE2 Expression in COVID-19 Gateway Tissues. Preprints 2020040315 (doi: 10.20944/preprints202004.0315.v1). It is proposed that the extracts can be used to develop easy-to-use preventative treatments in the form of mouthwash and throat gargle products.

Independent of COVID-19, cannabinoids and in particular cannabidiol have been considered as drugs. There is evidence that cannabinoids can be beneficial for treating a number of clinical conditions, including pain, inflammation, epilepsy, sleep disorders, indication of multiple sclerosis, anorexia, and schizophrenia (N. Bruni et al., Cannabinoid Delivery Systems for Pain and Inflammation Treatment. Molecules 2018, 23, 2478). While the use of cannabinoids in various indications has been suggested, so far only limited applications received market authorisation.

Data demonstrating utility of cannabinoids in the treatment of COVID-19 have so far not been disclosed.

### Summary of the Invention

An objective of the invention is to provide compositions and treatment regimens for the treatment of COVID-19 patients.

According to the invention there is provided cannabidiol, for the treatment of a patient suffering from an infection with SARS-CoV-2. Cannabidiol is administered as an antiviral agent and/or for preventing or ameliorating the cytokine release syndrome (CRS).

Cannabidiol reduces the viral load.

Further, the treatment reduces the serum IL-6 level. It also prevents or ameliorates the acute respiratory distress syndrome (ARDS).

Cannabidiol may be administered prophylactically.

The treatment of a patient may be initiated immediately after diagnosis of the disease, for instance, during the non-severe symptomatic period of COVID-19.

Treatment may be initiated if the patient has an increased IL-6 level.

Cannabidiol can be applied in combination with one or more antiviral agents, such as remdesivir (an inhibitor of the RNA polymerase of the virus) or ritonavir/lopinavir (an HIV medicament); in combination with a drug against idiopathic pulmonary fibrosis; or in combination with a drug against blood clots or a drug against cardiac arrhythmias.

Cannabidiol is preferably administered orally. It is administered at a dose between 150 mg and 5000 mg one to four times per day, for instance, between 250 mg and 5000 mg one to four times per day.

Cannabidiol can be formulated as a solid dispersion. The solid dispersion comprises the cannabinoid and a solubilizer which is an amphiphilic block copolymer capable of forming a micellar solution if combined with an aqueous medium.

The block copolymer is preferably a poloxamer.

The solid dispersion can further comprise a water-soluble film former.

Cannabidiol can also be incorporated in a formulation comprising a core and a coating on the core, wherein the coating comprises the cannabinoid, one or more water-soluble film formers and not more than 20 wt.-%, based on the weight of all components, other excipients.

Further objectives and their solution can be concluded from the detailed description of the invention below.

### Brief Description of the Figures

With reference to the figures the invention is explained in more detail below.
Fig. 1 schematically shows the preparation of a solid dispersion containing Cannabidiol and the interaction of the solid dispersion with aqueous media.
Fig. 2 shows the *in vitro* release from three pellet products comprising 2-[1R-3-methyl-6R-(1-methylethenyl)-2-cyclohexen-1-yl]-5-pentyl-1,3-benzenediol as active substance and low-viscosity hydroxypropylmethyl cellulose as film former.

### Detailed Description of the Invention

### Patients to Be Treated

The invention is defined in the appended claims.

The course of COVID-19 can in general be divided into three stages:
- I) asymptomatic incubation period (virus may already be detectable)
- II) non-severe symptomatic period (virus detectable)
- III) severe respiratory symptomatic stage

Early after infection, the immune response is essential to eliminate the virus and to prevent progression to the severe stage III. Strategies to boost immune responses at this stage may be important. Immunosuppressive therapies are expected to endanger the patient in this early disease phase. Treatments which reduce the viral load can prevent disease progression. Antiviral agents can be administered in this stage.

If the early immune response is impaired or insufficient, or if there is no effective antiviral treatment, the virus will propagate and then cause massive tissue damage, eventually leading to inflammation caused by pro-inflammatory cytokines. High virus load strongly affects and destroys tissue with high expression of angiotensin converting enzyme 2 (ACE2), the receptor for SARS-CoV-2. The damaged cells as a consequence result in innate inflammation largely mediated by pro-inflammatory macrophages and granulocytes. The lungs as well as other organs and tissues may be affected. ACE2 is highly expressed in lung and intestinal epithelia, but is also found in other tissues including heart, cardiovascular system and kidney.

In severe conditions, a cytokine release syndrome (CRS) is observed.

CRS can occur in a number of infectious and non-infectious diseases. CRS is a form of systemic inflammatory response syndrome. Immune cells are activated by stressed or infected cells through receptor-ligand interactions. CRS occurs when large numbers of white blood cells are activated to release inflammatory cytokines, which in turn activate more white blood cells in a positive feedback loop of pathogenic inflammation, leading to a rapid elevation of pro-inflammatory cytokines.

The term cytokine storm is used for severe cases of CRS.

In COVID-19, systemic hyperinflammation results in inflammatory lymphocytic and monocytic infiltration of the lung and the heart, causing ARDS and cardiac failure. Patients with fulminant COVID-19 and ARDS have classical serum biomarkers of CRS including elevated CRP, LDH, IL-6, and ferritin.

Patients requiring intensive care typically have higher blood concentrations of proinflammatory cytokines than those not requiring intensive care. A similar phenomenon was shown in a retrospective study with COVID-19 cases: the blood concentration of the proinflammatory cytokine IL-6 was significantly higher in patients who died from COVID-19 compared to disease survivors. Further, already after four days of illness onset, IL-6 concentrations were higher in non-survivors than in survivors. The IL-6 concentration curve of non-survivors is characterised by a steep increase immediately before their death, whereas the IL-6 concentration remained stable in survivors (F. Zhou et al. (2020). Clinical course and risk factors for mortality of adult inpatients with COVID-19 in Wuhan, China: a retrospective cohort study. Lancet 395(10229): 1054-62).

A high level of IL-6 is a hallmark and important driving force of the CRS.

CRS is considered to be the cause of several pathological events.

For instance, a relevant factor contributing to the lung pathology is a disturbed production and regulation of hyaluronan: cytokines are strong inducers of hyaluronan synthetase-2.

Hyaluronan has the ability to absorb water up to 1000 times of its molecular weight and therefore is assumed to be the underlying reason for the clear liquid jelly observed in the lungs of the severely affected patients.

In patients progressing to the severe stage III, lung inflammation is the main cause of acute respiratory distress syndrome (ARDS). The rapid onset of widespread inflammation in the lungs leads to respiratory failure. ARDS is a major cause of death from COVID-19.

Another main cause of death in patients with COVID-19 is circulatory failure due to myocardial injury. There are also reports of patients who died from fulminant myocarditis. Consistent with these findings, D-dimer levels >1 µg/mL and elevated high-sensitivity cardiac troponin I were associated with higher odds of in-hospital death in a retrospective study. In this study, more than half of the patients who died had increased cardiac troponin I and about 90 % of inpatients with pneumonia had increased D-dimer concentrations, indicative of high coagulation activity (F. Zhou *et al., loc. cit*.).

Thus, the release of pro-inflammatory cytokines that induce a procoagulant state and contribute to plaque rupture, predisposing patients to thrombosis and ischemia, contributes to the cardiac events in COVID-19 patients.

Further, the pathophysiological processes in COVID-19 patients are also reflected in the counts of certain white blood cells.

High white blood cell counts as well as lymphopenia and high neutrophil-to-lymphocyte ratios are common in COVID-19 patients (Y. Liu *et al.* (2020). Neutrophil-to-lymphocyte ratio as an independent risk factor for mortality in hospitalized patients with COVID-19. J Infect).

The available clinical data show that, while early during the course of the disease an immune response to the virus is essential, later on certain components of the immune response are actually damaging.

The present invention is based on the finding that pharmacological intervention can reduce the viral load and/or prevent or reduce unwanted components of the immune response. The invention relies on the administration of an active agent having a dual mode of action.

The invention in particular allows preventing or ameliorating the cytokine release syndrome (CRS) and its clinical manifestations, including unwanted inflammatory processes. This is achieved by a pharmacological intervention counteracting the release of pro-inflammatory cytokines, in particular IL-6.

Preliminary clinical data investigating the use of tocilizumab, a humanized monoclonal antibody against the IL-6 receptor, suggest beneficial effects of IL-6 blockade therapy in patients with severe SARS-CoV-2 pneumonia (X. Xu et al., Effective treatment of severe COVID-19 patients with tocilizumab. ChinaXiv:20200300026 (2020)).

The present invention provides a simpler and more convenient treatment, namely a treatment which can be administered orally. Moreover, according to the present invention, the active agent also has antiviral activity.

Furthermore, according to the present invention, treatment is started earlier, *i.e.*, before the severe stage of the disease is reached. It is in particular considered to start treatment at a point in time when CRS and its consequences can still be prevented or at least progression of CRS to severe stages can be halted or significantly slowed down.

This also means that more patients may benefit from the treatment as compared to approaches applying treatment only to severe cases.

According to the present invention, patients to be treated suffer from an infection with SARS-CoV-2. Confirmation of the infection can be determined by PCR.

Treatment may start upon hospitalization, but preferably is initiated in patients with confirmed SARS-CoV-2 infection if one or more of the criteria discussed below are met.

Patients in the symptomatic stage of the infection show symptoms of disease including, but not limited to, one or more of fever, dry cough, shortness of breath, and evidence of rales/crackles on physical examination, myalgia, fatigue, dyspnea, anorexia, loss of sense of smell and taste, and nephritis.

Thus, treatment may be initiated if a patient has been tested positive for SARS-CoV-2 and shows at least one of the symptoms listed above.

The pathological lung features of COVID-19 include ground glass opacities, crazy-craving pattern and in later stages consolidation on chest computed tomography (CT) or chest x-ray.

Treatment may be initiated if a patient has been tested positive for SARS-CoV-2 and shows pathological lung features either by CT-scan or chest x-ray.

Treatment may be initiated based on the saturation of peripheral oxygen (SpO2).

Treatment may be initiated if a patient has been tested positive for SARS-CoV-2 and shows reduced saturation of peripheral oxygen (SpO2). In particular, treatment may be initiated if a patient shows a saturation of peripheral oxygen (SpO2) of ≤93% at rest in ambient air or requires between 3L/min and 5L/min of oxygen to maintain SpO2 >97%.

Further, treatment of a patient who has been tested positive for SARS-CoV-2 may be initiated upon worsening of lung involvement, defined as worsening of oxygen saturation >3 percentage points or decrease in PaO2 (partial pressure of oxygen, arterial) >10%, with stable FiO2 (fraction of inspired oxygen) in the last 24h.

Patients may also be treated at the beginning of NIV (non-invasive ventilation) or CPAP (continuous positive airway pressure), although an earlier treatment start is preferable. Suitable criteria for initiating treatment may also be based on laboratory findings.

Laboratory findings upon which treatment of a patient who has been tested positive for SARS-CoV-2 may be initiated include one or more of serum IL-6 ≥ 5.4 pg/ml; CRP level >70 mg/L (without other confirmed infectious or non-infectious course); CRP level >= 40 mg/L and doubled within 48 hours (without other confirmed infectious or non-infectious course); lactate dehydrogenase > 250 U/L; D-dimer > 1 µg/mL; serum ferritin > 300 µg/mL. Preferably, treatment initiation is based on an increased level of IL-6.

Optionally, treatment is initiated if the patient who has been tested positive for SARS-CoV-2 shows at least one of the above symptomatic criteria and meets at least one of the above laboratory criteria.

Further, treatment of a patient who has been tested positive for SARS-CoV-2 may be initiated if the patient, optionally in addition to one of the above criteria, shows thrombocytopenia < 120.000 x 10E9/L, and/or a lymphocyte count < 0.6 x 10E9/L. Patients treated may belong to a risk group. For instance, patients treated may suffer from adipositas. In particular, patients treated may suffer from adipositas and have a serum IL-6 level ≥ 5.4 pg/ml.

Treatment progress can be monitored by reduction of IL-6, CRP, transaminases, LDH, D-dimer, ferritin, IL-1β, IL-18, interferon gamma, neutrophils, lymphocytes, neutrophil-to-lymphocyte ratio (NLR) in %, for instance between first dose, day 14 and day 28.

The treatment is continued until relevant clinical improvements are achieved, for instance, until independence from supplementary oxygen therapy or until resolution of fever. Clinical efficacy can be confirmed by overall clinical improvement; the prevention of invasive ventilation in patients with moderate COVID-19; the improvement of laboratory parameters indicative of disease severity.

According to the invention, cannabidiol can also be used for the treatment of a subject at risk to be infected with SARS-CoV-2 (prophylactic administration). A prophylactic administration is in particular based on the antiviral activity of the cannabinoid.

Efficacy of prophylaxis may be assessed by the absence of a viral load or a reduced viral load in the subject after exposure to SARS-CoV-2; by an asymptomatic course of the disease or by a reduced severity of the disease compared to subjects not prophylactically treated.

### Active Ingredients

Cannabinoids are a heterogeneous group of pharmacologically active substances that have an affinity for the so-called cannabinoid receptors. The cannabinoids include, for example, tetrahydrocannabinol (THC) and the non-psychoactive cannabidiol (CBD). Cannabinoids can be both phytocannabinoids and synthetic cannabinoids. Phytocannabinoids are a group of about 70 terpenophenolic compounds (V.R. Preedy (ed.), Handbook of Cannabis and Related Pathologies (1997)). These compounds typically contain a monoterpene residue that is attached to a phenolic ring and has a C₃-C₅ alkyl chain that is in the meta position to the phenolic hydroxyl group.

A group of cannabinoids are tetrahydrocannabinols with the following general formula (1): 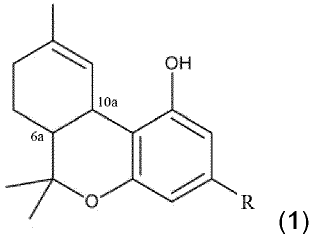 wherein R is selected from among C₁-C₂₀-alkyl, C₂-C₂₀-alkenyl or C₂-C₂₀-alkynyl, and optionally has one or more substituents.

In a further preferred group of compounds of the above general formula (1), R is selected from among C₁-C₁₀-alkyl or C₂-C₁₀-alkenyl, and optionally has one or more substituents. In particular, in formula (1) R is an alkyl radical with the formula C₅H₁₁.

Compounds of general formula (1) can be present in the form of stereoisomers. The centres 6a and 10a preferably each have the R configuration.

The tetrahydrocannabinol is in particular Δ9-THC with the chemical name (6aR,10aR)-6,6,9-trimethyl-3-pentyl-6a, 7,8,10a-tetrahydro-6H-benzo[c]chromene-1-ol. The structure is reflected by the following formula (2):

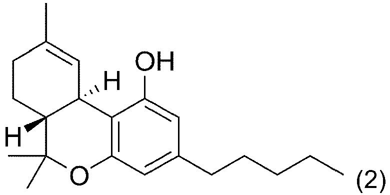

Another group of cannabinoids are cannabidiols with the following general formula (3): 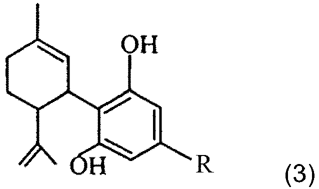 wherein R is selected from among C₁-C₂₀-alkyl, C₂-C₂₀-alkenyl or C₂-C₂₀-alkynyl, and optionally has one or more substituents.

In a further preferred group of compounds having the general formula (3) above, R is selected from among C₁-C₁₀-alkyl or C₂-C₁₀-alkenyl, and optionally has one or more substituents.

In particular, R in formula (3) is an alkyl radical with the formula C₅H₁₁.

The cannabidiol is in particular 2-[(1R,6R)-3-methyl-6-(1-methylethenyl)-2-cyclohexen-1-yl]-5-pentyl-1,3-benzenediol. In the present specification, if the term cannabidiol or its abbreviation CBD is used, this particular compound is meant, unless stated otherwise. CBD is a major constituent of *Cannabis sp.* - besides the psychotropic Δ9-THC. The psychotropic effect of THC is mediated by the cannabinoid receptor CB1 that is mainly expressed on neurons. In contrast to THC, CBD is a peripherally and centrally acting compound without psychotropic activity.

According to the invention, a combination of Δ9-THC ((6aR, 10aR)-6,6,9-trimethyl-3-pentyl-6a,7,8,10a-tetrahydro-6H-benzo[c]chromen-1-ol) and CBD (2-[(1R,6R)-3-methyl-6-(1-methylethenyl)-2-cyclohexen-1-yl]-5-pentyl-1,3-benzenediol) can be used.

Another group of cannabinoids are cannabinols with the following general formula (4): 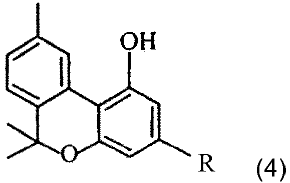 wherein R is selected from among C₁-C₂₀-alkyl, C₂-C₂₀-alkenyl or C₂-C₂₀-alkynyl, and optionally has one or more substituents.

In a further preferred group of compounds having the general formula (4) above, R is selected from among C₁-C₁₀-alkyl or C₂-C₁₀-alkenyl, and optionally has one or more substituents.

In particular, in formula (4) R is an alkyl radical having the formula C₅H₁₁.

The cannabinol is especially 6,6,9-trimethyl-3-pentyl-6*H*-dibenzo[*b*,*d*]pyran-1-ol.

In some embodiments, cannabinoids or cannabinoid mixtures of hemp extracts can also be used.

For example, Nabiximols is a plant extract mixture used as a drug of the leaves and flowers of the hemp plant (Cannabis sativa L.) with standardized contents of tetrahydrocannabinol (THC) and cannabidiol (CBD).

Synthetic cannabinoids can also be used.

These include 3-(1,1-dimethylheptyl)-6,6a,7,8,10,10a-hexahydro-1-hydroxy-6,6-dimethyl-9*H*-dibenzo[*b*,*d*]pyran-9-one. This compound contains two stereogenic centres. The drug nabilone is a 1:1 mixture (racemate) of the (6a*R*,10a*R*) form and the (6a*S*,10a*S*) form. Nabilone is a cannabinoid.

Another example of a synthetic cannabinoid is JWH-018 (1-naphthyl-(1-pentylindol-3-yl)methanone).

The use of cannabinoids, in particular of cannabidiol, is based on their pharmacodynamic properties. Cannabinoid receptors include CB1, which is predominantly expressed in the brain, and CB2, which is primarily found on the cells of the immune system. The fact that both CB1 and CB2 receptors have been found on immune cells suggests that cannabinoids play an important role in the regulation of the immune system. Independent of this finding, several studies show that cannabinoids downregulate cytokine and chemokine production and, in some models, upregulate T-regulatory cells (Tregs) as a mechanism to suppress inflammatory responses. The endocannabinoid system is also involved in immunoregulation.

Cannabinoids, in particular cannabidiol, are in particular suitable for preventing CRS in COVID-19 patients or at least halting or significantly slowing down progression of CRS to severe stages in COVID-19 patients.

This therapeutic utility is based on the pharmacodynamic properties of the cannabinoids, especially their interaction with the endocannabinoid system and further pharmacological targets including serotonergic receptors, adenosine signalling, vanilloid receptors, PPAR-γ receptors and GPR55, which has been shown to be immune-modulating or even immune-suppressive.

Cannabinoids, in particular cannabidiol, exert effects on the innate immune system (i.e., the part of the immune system enabling a fast reaction to pathogens via neutrophils, macrophages and other myeloid cells). Affected cell types of the innate immune system in particular include mononuclear cells, macrophages, neutrophils, dendritic cells, microglial cells and myeloid-derived suppressor cells (MDSCs) (J.M. Nichols and B.L.F. Kaplan (2020). Immune responses regulated by cannabidiol. Cannabis and Cannabinoid Research 5(1): 12-31):
- The release of pro-inflammatory cytokines in human mononuclear cells is suppressed by nanomolar or micromolar concentrations of CBD.
- CBD (20 mg/kg) decreases the number of leukocytes including macrophages and neutrophils in the bronchoalveolar lavage fluid of mice after LPS-induced lung inflammation. This effect is mediated by the adenosine A2A receptor (A. Ribeiro et al. (2012). Cannabidiol, a non-psychotropic plant-derived cannabinoid, decreases inflammation in a murine model of acute lung injury: role for the adenosine A(2A) receptor. Eur J Pharmacol 678(1-3): 78-85). Furthermore, CBD also inhibits the migration of human neutrophils (D. McHugh et al. (2008). Inhibition of human neutrophil chemotaxis by endogenous cannabinoids and phytocannabinoids: evidence for a site distinct from CB1 and CB2. Mol Pharmacol 73(2): 441-50). Reduction in neutrophil count is of therapeutic relevance in patients with COVID-19 as a high neutrophil-to-lymphocyte ratio has been shown to be an independent risk factor of mortality in these patients (Y. Liu *et al., loc. cit*.).
- CBD suppresses the CD83 dendritic cell activation marker on dendritic cells derived from individuals with human immune deficiency virus (HIV) infection, but not healthy individuals (A.T. Prechtel and A. Steinkasserer (2007). CD83: an update on functions and prospects of the maturation marker of dendritic cells. Arch Dermatol Res 299(2): 59-69).
- CBD (1-16 µmol/l) induces apoptosis in microglial cells, the main innate immune cells of the central nervous system (H.Y. Wu et al. (2012). Cannabidiol-induced apoptosis in murine microglial cells through lipid raft. Glia 60(7): 1182-90).
- The numbers of natural killer (NK) cells and natural killer T (NKT) cells are not affected by CBD (5 mg/kg per day) or even increased (2.5 mg/kg per day) in healthy rats, suggesting that CBD may enhance the NK/NKT-related non-specific immune response (B. Ignatowska-Jankowska et al. (2009). Cannabidiol-induced lymphopenia does not involve NKT and NK cells. J Physiol Pharmacol 60 Suppl 3: 99-103).
- Additionally, CBD is able to induce the regulatory immune cell population of MDSCs. In mice with chemically induced acute hepatitis, CBD (25 mg/kg) induces the expression of MDSCs, along with a reduction of pro-inflammatory cytokines such as IL-2, TNF-α and IL-6; the effect is mediated by the TRPV1 receptor (V.L. Hegde et al. (2011). Role of myeloid-derived suppressor cells in amelioration of experimental autoimmune hepatitis following activation of TRPV1 receptors by cannabidiol. PLoS One 6(4): e18281).

In addition, cannabinoids, in particular CBD, exhibit an effect on cells of the adaptive immune system. The adaptive immune system is comprised of T and B cells. T cells either directly lyse or induce apoptosis of infected cells (cytotoxic T cells) or recruit other immune cells (T helper [Th] cells) including B cells that produce antibodies against pathogens:
- In a study with healthy rats, daily administration of 5 mg/kg CBD significantly reduced the number of T cells including T helper cells and cytotoxic T cells and of B cells (B. Ignatowska-Jankowska *et al., loc. cit*.).
- It has been suggested that a shift from Th1 to Th2 immune response resulting in decreased pro-inflammatory cytokines such as TNF-α and IL-12 and increased anti-inflammatory cytokines such as IL-10 accounts for CBD's anti-inflammatory effects (L. Weiss et al. (2006). Cannabidiol lowers incidence of diabetes in nonobese diabetic mice. Autoimmunity 39(2): 143-51).
- In an activated memory T cell line, CBD dose-dependently (1-5 µmol/l) reduced the autoantigen-specific Th17 cell phenotype as shown by a decrease of the Th17 signature cytokine IL-17. The finding was accompanied by decreased IL-6 production and secretion and increased production of IL-10, critical changes associated with reduced Th17 cell propagation (E. Kozela et al. (2013). Cannabinoids decrease the th17 inflammatory autoimmune phenotype. J Neuroimmune Pharmacol 8(5): 1265-76). These results are especially relevant with respect to COVID-19 as pathological findings of a patient who died from COVID-19 included an increased Th17 cell proportion (Z. Xu et al. (2020). Pathological findings of COVID-19 associated with acute respiratory distress syndrome. Lancet Respir Med 8(4): 420-2).
- CBD was shown to induce regulatory T cells (Tregs) in several disease models (J.M. Nichols and B.L.F. Kaplan (2020), *loc. cit*.). In mice with ischemia-reperfusion-induced kidney injury, levels of regulatory T-17 (Treg17) cells were decreased and Th17 levels were increased. The physiological function of Treg17 cells includes the inhibition of Th17-mediated inflammatory actions. A dose of 10 mg/kg CBD after induced kidney injury was renoprotective and reversed these effects (B. Baban et al. (2018). Impact of cannabidiol treatment on regulatory T-17 cells and neutrophil polarization in acute kidney injury. Am J Physiol Renal Physiol 315(4): F1149-f58). These results further support the beneficial effect of CBD in COVID-19.

Many studies demonstrate that cannabinoids and in particular CBD exert their immune suppressive and anti-inflammatory effects by the suppression of pro-inflammatory cytokines such as TNF-α, IFN-γ, IL-6, IL-1β, IL-2, IL-17A, and of chemokines, such as CCL-2. The pro-inflammatory cytokine IL-6 has a central role in the cytokine release syndrome (CRS) in patients with severe COVID-19 and IL-6 signalling is among the main canonical pathways affected by cannabinoids and in particular CBD. Since cannabinoids and in particular CBD suppress circulating IL-6 in various inflammation animal models including a model of acute lung injury, suppression of IL-6 thereby preventing the CRS is considered the most relevant mode of action of cannabinoids and in particular CBD in patients with COVID-19.

An *in vitro* cell culture study suggests that certain *Cannabis sativa* extracts down-regulate ACE2, the receptor for SARS-CoV-2, and also down-regulate serine protease TMPRSS2, another critical protein required for SARS-CoV-2 entry into host cells (B. Wang *et al., loc. cit*.). This suggests that cannabinoids may have additional beneficial effects when administered to COVID-19 patients.

According to the present invention, cannabidiol can also be applied as part of a combination treatment.

Cannabidiol can be administered in combination with one or more antiviral agents. Antiviral drugs that may be used for the combination therapy are those that were originally developed for HIV, Ebola, hepatitis C, flu, SARS, or MERS (two of other coronavirus diseases). They are designed to block the multiplication of viruses or prevent them from entering human cells.

In one aspect, cannabidiol is used in combination with remdesivir (an inhibitor of the RNA polymerase of the virus). In another aspect, cannabidiol is used in combination with ritonavir/lopinavir (an HIV medicament).

Cannabidiol can also be used in combination with medicines for lung patients, that were developed against idiopathic pulmonary fibrosis preventing the patient's lungs from being able to supply the blood with enough oxygen.

Further, cannabidiol can be used in combination with cardiovascular drugs, in particular drugs against blood clots or cardiac arrhythmias.

### Dosing and Administration

According to the invention, cannabidiol is preferably administered orally.

Other routes of administration are, however, also contemplated, in particular for patients who cannot take an oral medication. Such other routes are in particular intravenous, intramuscular or subcutaneous injection.

The administration is in one to four doses per day. Typically, the administration is twice per day (BID).

According to the invention, patients are treated with an effective dose of cannabidiol.

A single dose may be between 150 mg and 5000 mg, such as between 250 mg and 5000 mg, administered one to four times per day, for instance, BID.

Exemplary doses are 375 mg, 750 mg, 1500 mg, and 3000 mg, administered one to four times per day, for instance, BID.

A particularly preferred dose is 1500 mg, administered one to four times per day, preferably, BID.

As indicated above, cannabinoids, in particular cannabidiol, have antiviral activity and have suppressive pharmacodynamic effects on the immune system in various animal models. It has been shown in divergent animal models that in the majority of cases inflammatory processes are suppressed by doses between 2.5 and 20 mg/kg body weight mostly administered intraperitoneally or orally. Alternative routes have been transdermal, intranasal and IV application (J.M. Nichols and B.L.F. Kaplan BLF (2020), *loc. cit*.).

In cellular models determining a suppressive effect on IL-6 secretion in the majority of cases the effective concentration was in a magnitude of 5 µM (J. Chen et al. (2016). Protective effect of cannabidiol on hydrogen peroxide-induced apoptosis, inflammation and oxidative stress in nucleus pulposus cells. Mol Med Rep 14(3): 2321-7).

Based on the molecular weight of CBD of 314.5 g/mol the resulting concentration is 1,570 ng/ml.

Ribeiro *et al.* investigated the influence of CBD on LPS-induced acute lung injury in mice as disease model for ARDS, once in a prophylactic intervention (A. Ribeiro *et al.* (2012), *loc. cit*.) and once in the acute phase as a therapeutic intervention (A. Ribeiro et al. (2014). Cannabidiol improves lung function and inflammation in mice submitted to LPS-induced acute lung injury. Immunopharmacol Immunotoxicol 37(1): 35-41). ARDS plays a major role in the pathological scenario of COVID-19.

Mice were prophylactically administered 0.3, 1.0, 10, 20, 30, 40 and 80 mg/kg CBD via the intraperitoneal route. 60 minutes after administration acute lung injury was induced via intranasal instillation of Escherichia coli LPS. Mice were killed 1, 2, 4 and 7 days after instillation. Total leukocytes migration, myeloperoxidase activity, pro-inflammatory cytokine production including TNF-α and IL-6 and vascular permeability were significantly decreased (A. Ribeiro *et al.* (2012), *loc. cit*.). Effects were dose dependent but reached a nearly maximum extent with 20 mg/kg in this study with prophylactic application.

In a subsequent study the same group investigated the effect of CBD after acute lung injury had been induced by LPS. The testing scenario was similar except for the time point of intervention which was chosen as 6h after LPS installation. Doses of 20 and 80 mg/kg were chosen based on the results of the earlier study (A. Ribeiro *et al.* (2014), *loc*. *cit*.). The study showed an improved mechanical lung function, decreased leukocyte migration (neutrophil, macrophages and lymphocytes) into the lungs, decreased myeloperoxidase activity in the lung tissue, reduced vascular permeability and production of proinflammatory cytokines/chemokines at 20 mg/kg.

A comparative investigation for systemic exposure after i.p. and oral application of CBD in mice and rats has shown that 120 mg/kg as a single dose leads to a maximum plasma concentration of 14,000 ng/ml in mice (S. Deiana et al. (2012). Plasma and brain pharmacokinetic profile of cannabidiol (CBD), cannabidivarine (CBDV), Delta(9)-tetrahydrocannabivarin (THCV) and cannabigerol (CBG) in rats and mice following oral and intraperitoneal administration and CBD action on obsessive-compulsive behaviour. Psychopharmacology (Berl) 219(3): 859-73).

Taking these data into consideration and assuming a dose-proportional relationship for the resulting plasma concentrations, a dose of 20 mg/kg, shown to be effective in the animal model, leads to a target peak exposure of 2,300 ng/ml.

As regards systemic exposure data in humans, after fasted administration of Epidyolex^{®} morning maximum values under steady-state conditions of 541 ng/ml are observed. Evening maximum values are higher. A factor of 3.8 in systemic exposure is observed between morning and evening upon twice daily Epidyolex^{®} administration (L. Taylor et al. (2018). A Phase I, Randomized, Double-Blind, Placebo-Controlled, Single Ascending Dose, Multiple Dose, and Food Effect Trial of the Safety, Tolerability and Pharmacokinetics of Highly Purified Cannabidiol in Healthy Subjects. CNS Drugs 32(11): 1053-67).

Thus, the standard dose of 1,500 mg CBD administered twice daily as already approved with Epidyolex^{®} is considered safe and efficacious.

Based on the above data, patients will also benefit from other doses in the range outlined herein.

### Galenics

Low and variable bioavailability of cannabinoids, in particular upon oral administration, hampers effective clinical use of these compounds.

Cannabinoids, in particular cannabidiol, are difficult to formulate due to their highly lipophilic nature.

In fact, cannabinoids are highly lipophilic molecules (log P 6-7) with very low water solubility (2-10 µg / ml). The log P is the decimal logarithm of the n-octanol/water partition coefficient. The partition coefficient can be determined experimentally. Values typically refer to room temperature (25°C). The partition coefficient can also be roughly calculated from the molecular structure.

In addition to poor solubility, cannabinoids, in particular CBD, are subject to high first-pass metabolism, which further contributes to poor systemic availability after oral administration.

Various formulations of cannabinoids have been suggested.

Due to the high lipophilicity of cannabinoids, salt formation (*i.e*. pH adjustment), cosolvency (e.g. ethanol, propylene glycol, PEG400), micellization (*e.g*. Polysorbate 80, Cremophor-ELP), emulsification including micro and nano emulsification, complexation (*e.g.* cyclodextrins) and encapsulation in lipid-based formulations (*e.g*. liposomes) are among the formulation strategies considered in the prior art. Nanoparticle systems have also been proposed (N. Bruni *et al., loc. cit*.).

Various solid oral dosage forms have been proposed in the patent literature, for example in WO 2008/024490 A2 and in WO 2018/035030 A1. These documents do not contain data on release behaviour, so the practical suitability of the proposed forms for the administration of cannabinoids remains unclear.

WO 2015/065179 A1 describes compressed tablets which, in addition to cannabidiol, contain lactose and sucrose fatty acid monoesters.

Dronabinol (Δ9-THC) is marketed in the form of capsules (Marinol^{®}) and as an oral solution (Syndros^{®}). The Marinol^{®} capsules are soft gelatine capsules containing the active ingredient in sesame oil.

The drug product Sativex^{®} containing nabiximols is a mouth spray that is sprayed onto the inside of the cheek.

Self-emulsifying drug delivery systems (SEDDS) which are mixtures of oils, surfactants and optionally contain hydrophilic solvents have also gained interest in an approach to improve the oral bioavailability of certain cannabinoids (K. Knaub et al. (2019). A Novel Self-Emulsifying Drug Delivery System (SEDDS) Based on VESIsorb® Formulation Technology Improving the Oral Bioavailability of Cannabidiol in Healthy Subjects. Molecules, 24(16), 2967). Upon contact with an aqueous phase, such as gastric or intestinal fluids, SEDDS spontaneously emulsify under conditions of gentle agitation.

VESIsorb^{®}, a self-emulsifying drug delivery formulation technology developed by Vesifact AG (Baar, Switzerland) has shown increased oral bioavailability of certain lipophilic molecules.

The preparation Epidiolex^{®} recently approved by the US-FDA as an orphan drug for the treatment of certain forms of epilepsy is provided in the form of an oral solution that in addition to the active ingredient cannabidiol contains the excipients absolute ethanol, sesame oil, strawberry aroma and sucralose.

Notwithstanding all these proposals, however, there is still a need for improved dosage forms for cannabinoids, such as cannabidiol, in particular for solid oral dosage forms.

Various approaches suggested in the prior art are not entirely satisfactory. Some of these approaches rely on liquid formulations. Handling of such formulations is more difficult than that of solid dosage form. Prior art formulations are often complex to prepare and sometimes lead to only low bioavailability of the cannabinoid.

While formulations known in the art may be used in the treatment aspects of the present invention, the invention also provides improved formulations.

It is to be understood that these formulations are not only useful in the context of the treatment aspects of the present invention but constitute a contribution as such. The formulations disclosed herein may be used for any treatment for which the use of the contained active ingredient in indicated.

In one aspect of the present invention, a formulation is provided which is a solid dispersion comprising cannabidiol and a solubilizer. As further detailed below, solid dosage forms for oral administration showing satisfactory bioavailability can be obtained in this way.

According to this aspect, a highly lipophilic cannabinoid, like the almost water insoluble CBD, is combined with a solubilizer in order to increase the drug solubility by solubilization in aqueous media. An increased solubility will in turn increase the absorption rate of the drug compound.

Preferably, no toxic or otherwise harmful degradation products are formed during preparation or storage of the formulations.

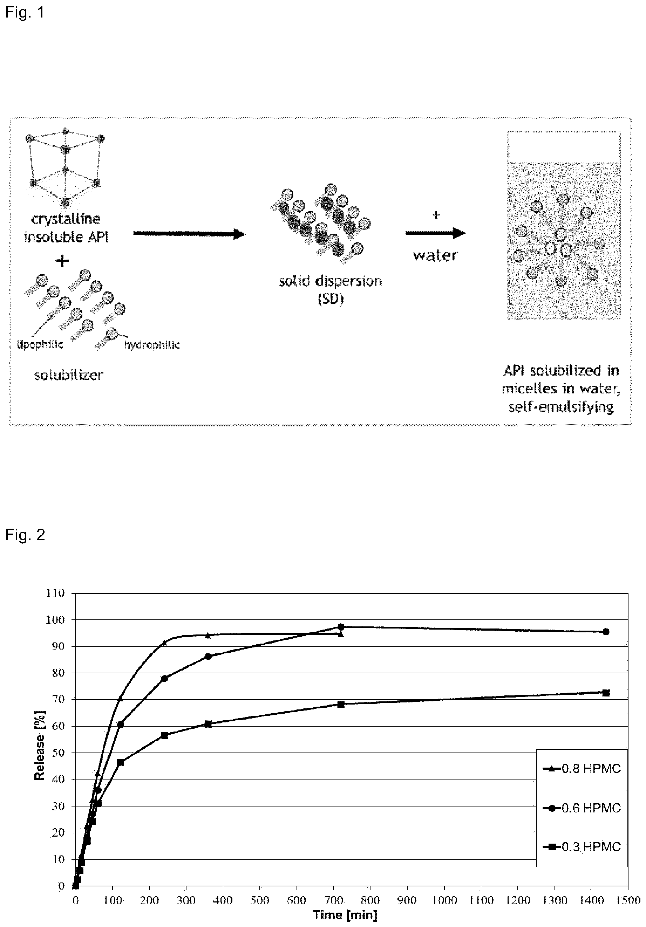

The solid dispersion comprising cannabidiol and a solubilizer leads to the formation of micelles upon contact with water or other aqueous media, such as gastrointestinal fluids. The micelles are essentially formed from the drug substance, surrounded by solubilizer (see Fig. 1).

One aspect of the invention is accordingly a micellar composition comprising an aqueous phase in which micelles are dispersed, which micelles comprise cannabidiol and a solubilizer.

Suitable solubilizers are solid at ambient temperature. They have surfactant properties and, if used in appropriate concentration ranges in aqueous media, in particular water, can form micellar solutions.

Suitable solubilizers include in particular amphiphilic block copolymers.

More in particular, block copolymers containing at least one polyoxyethylene block and at least one polyoxypropylene block can be used.

Suitable block copolymers are in particular poloxamers. Poloxamers are block copolymers whose molecular weights range from 1,100 to over 14,000. Different poloxamers differ only in the relative amounts of propylene and ethylene oxides added during manufacture. Poloxamers have the following general formula:

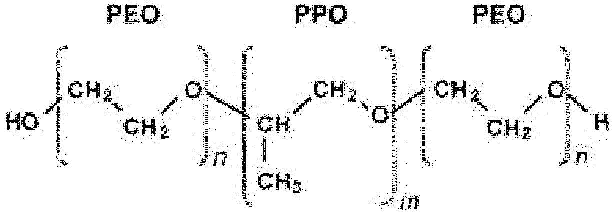

In this general formula, n designates the number of polyoxyethylene units, m designates the number of polyoxypropylene units.

In one embodiment, the solubilizer is Poloxamer 188 (Kolliphor P188; former brand name Lutrol F 68) / BASF; CAS No.: 9003-11-6).

Kolliphor P188 is a polyoxyethylene-polyoxypropylene block copolymer of the above general formula wherein n is approximately 79 and m is approximately 28.

Kolliphor P188 is available as a white to slightly yellowish waxy substance in the form of micropearls having a melting point of 52 - 57°C. It meets the requirements of Ph.Eur., USP / NF for Poloxamer 188.

The cannabinoid and the solubilizer are present in a weight ratio cannabinoid: solubilizer of typically 1:0.2 - 10.0, preferably 1:0.5 - 6.0, in particular 1:1 - 5.

The solid dispersion according to the above formulation aspect of the invention can be prepared by a hot melt process. The cannabinoid and the solubilizer are heated to a temperature which allows forming a homogenous melt in which the cannabidiol and the solubilizer are present in a molecular state before they form a solid dispersion when cooled.

The melt is processed into pellets. This can be carried out by batch-wise spray granulation / pelletisation (fluid bed topspray, Wurster = bottomspray technology).

Alternatively, and preferably, continuous spray granulation / pelletisation (fluid bed MicroPx Technology, ProCell Technology) is used.

An alternative preparation method relies on dispersing the cannabinoid, in particular cannabidiol, in an aqueous solution of the solubilizer, for instance, in a solution of the solubilizer in water.

The solution can be processed by batch-wise spray granulation / pelletisation (fluid bed topspray or Wurster = bottomspray technology) or preferably by continuous spray granulation / pelletisation (fluid bed MicroPx Technology, ProCell Technology) to obtain a solid granulate.

The formulation may contain one or more excipients in addition to the active ingredient and the solubilizer. It is in particular considered to include an antioxidant or a combination of antioxidants to protect the cannabinoid, in particular cannabidiol, from oxidation.

Cannabinoids, in particular cannabidiol, are susceptible to oxidation. For instance, cannabidiol can be oxidized to monomeric and dimeric hydroxyquinones. The oxidation can lead to discoloration.

The oxidation can not only occur by molecular oxygen, but also by peroxides which may be introduced into the formulation by one or more of the excipients used.

Useful antioxidants which may be included into the formulations encompass ascorbyl palmitate, alpha-tocopherol, butylhydroxytoluol (BHT, E321), butylhydroxyanisol (BHA, E320), ascorbic acid, and ethylenediaminetetraacetic acid (EDTA) sodium.

Ascorbyl palmitate is a preferred antioxidant. It can effectively suppress discoloration by oxidation.

The antioxidant or combination of antioxidants may be added to the melt or the solution of the solubilizer prior to the addition of the cannabinoid, in particular CBD.

The antioxidant is typically used in an amount of 0.5 to 2.5 wt%, preferably of 0.8 to 2 wt%, in particular 1.0 to 1.8 wt%, relative to the amount of the cannabinoid (in particular cannabidiol).

The solid dispersion preferably does not contain more than 20 % by weight, relative to all components, of additional excipients.

The solid dispersion is preferably free or essentially free of triglycerides. Essentially free means that the formulation contains less than 5 % by weight, relative to all components, of triglycerides.

Further, the solid dispersion is preferably free or essentially free of fatty acids. Essentially free means that the formulation contains less than 5 % by weight, relative to all components, of fatty acids.

Preferably, the total amount of mono-, di- and triglycerides and fatty acids is less than 5 % by weight, relative to all components.

The solid dispersion granules or pellets can be filled into hard gelatine capsules, sachets or stick packs using commercial standard technology and equipment.

Depending on the final dosage strength per unit, the solid dispersion granules can be filled into capsules which are feasible for swallowing (*e.g.* capsule size 2-1 for 25 mg/dose). Alternatively, for high dosed units, bigger capsules can be used as a primary packaging material for the granules. Such capsules are not for swallowing (*e.g*. capsule size up to 000 / sprinkle caps for 100-200 mg/dose). Rather, the solid dispersion granules are to be sprinkled on food or dispersed in a liquid, *e.g.*, water.

A composition obtained by dispersing the solid dispersion granules in a liquid can be applied to patients being not able to swallow by means of a syringe through a gastric tube. Alternatively, the solid dispersion granules can also be processed into tablets. The solid dispersion granules are combined with one or more excipients, such as a disintegrant, a glidant, and/or a lubricant. The obtained mixture is then compressed into tablets.

According to another aspect of the invention a product for the release of cannabidiol, comprises a core and a coating on the core, wherein the coating comprises the cannabidiol, one or more highly lipophilic physiologically active substances, one or more water-soluble film formers and no more than 20 wt.-% of other excipients, based on the weight of all components.

Preferably, no toxic or otherwise harmful degradation products are formed during preparation or storage of the formulations.

Surprisingly, it was found that solid oral dosage forms of cannabidiol, can be provided, wherein the release can be controlled with the help of the amount of film-forming agent(s) relative to the amount of the cannabinoid.

The use of one or more film formers not only allows for the formation of a coating containing cannabidiol, but also serves to control the release. In particular, a film former promotes the release of the cannabinoids which are only sparingly soluble in water. By means of the film former, these are released in sufficient quantity and speed.

For this purpose, a core is provided with a coating which, in addition to cannabidiol, comprises one or more water-soluble film formers. In addition to the cannabidiol, the coating preferably does not contain any other physiologically active substances.

Examples of suitable water-soluble film formers are methyl cellulose (MC), hydroxypropyl methyl cellulose (HPMC), hydroxypropyl cellulose (HPC), hydroxyethyl cellulose (HEC), sodium carboxymethyl cellulose (Na-CMC) and polyvinyl pyrrolidone (PVP). Hydroxypropylmethyl cellulose (HPMC), in particular low-viscosity HPMC, such as HPMC with a viscosity of a 2% (w/w) aqueous solution at 20°C of 6 mPa·s or less is preferred. An HPMC with a viscosity of a 2% (w/w) aqueous solution at 20°C of 3 mPa·s, as is available under the trade name Pharmacoat^{®} 603, is especially preferred.

The coating of cannabidiol and one or more water-soluble film formers may contain other commonly used excipients. According to the invention, the quantity of further excipients is limited to not more than 20 wt.-%, based on the weight of all components. Preferably, no more than 10 wt.-%, based on the weight of all components, of further excipients is comprised.

In a particularly preferred embodiment, the coating consists of cannabidiol and film former(s).

Pellets according to the invention have a coating which contains one or more water-soluble film formers, based on the total amount of cannabidiol, in a total amount of 0.1-10 wt.-%, preferably in a total amount of 0.5-8 wt.-%, and in particular in a total proportion of 1-6 wt.-%.

It is assumed that if the amount of film former is too small, the release takes place only very slowly and incompletely. By selecting a proportion in the specified ranges the release of the physiologically active substance can be adjusted. For example, the release from an oral dosage form can be adjusted so that the physiologically active substance is released over the conventional time of the gastrointestinal passage.

The coating is applied to cores. The cores may have any structure and may consist of any physiologically acceptable materials. For example, tablets, mini-tablets, pellets, granules or crystals may be used as cores. The cores may contain or consist of, for example, sugar, tartaric acid or microcrystalline cellulose. Inert starter cores, such as pellets made of microcrystalline cellulose, are preferred. Such pellets are commercially available under the name Cellets^{®}.

The size of the cores is not limited. Suitable sizes are in the range from 10 µm to 2000 µm, for example in the range from 50 µm to 1500 µm and preferably 100 µm to 1000 µm, the size may be determined by sieve analysis. In particular, pellets from a sieve fraction of 500-710 µm may be used.

The products according to the present aspect of the invention can be produced by first producing a spray liquid which contains cannabidiol and one or more water-soluble film formers.

Since cannabinoids have only a very low solubility in water, an organic solvent or a mixture of an organic solvent and water is typically used.

The spray liquid is then applied to cores. The liquid components are evaporated, so that a coating is formed on the cores that is mostly free of solvents and water. This may be done, for example, in a fluidized bed system, a jet bed system, a spray dryer or a coater.

Coated cores may then be used as an oral dosage form. Coated pellets may, *e.g.,* be offered in sachets, or they may be processed further.

The cores coated according to the present aspect of the invention may also be provided with one or more further coatings. This enables additional control of the release.

In a preferred embodiment, no further coating controlling the release is provided. Coated pellets may also be used to obtain multiparticulate dosage forms. For example, they can be filled into capsules or incorporated into tablets. In one embodiment, they are processed into orally dispersible tablets.

Coated pellets with different release profiles may be combined in one dosage form (capsule/tablet/sachet). The products according to this aspect of the invention release the cannabinoid contained therein after ingestion in the digestive tract. The products are especially used for controlled release. They in particular release more than 30 wt.-% and less than 80 wt.-% of the physiologically active substance contained within two hours. In addition, they, especially, release more than 40 wt.-% and less than 90 wt.-% of the physiologically active substance contained within three hours. Furthermore, they release more than 50 wt.-% and less than 95 wt.-% of the physiologically active substance contained within four hours. If more than one cannabinoid is comprised, the information relates to all substances contained.

In each case the release is determined in a blade stirrer apparatus in 1000 ml of phosphate buffer pH 6.8 with an addition of 0.4% Tween^{®} 80 at 37°C.

According to a further formulation approach of the invention, a solid dosage form is provided wherein the release rate of the cannabidiol can be adjusted by incorporating a combination of a solubilizer and a water-soluble film former into the formulation. In such a formulation, the water-soluble film former acts as a polymeric binder and additional solubilizer. The formulation is in the form of a solid dispersion.

Solid dosage forms for oral administration showing satisfactory bioavailability can be obtained in this way. Dosage forms according to the present invention also show a reduced food effect.

Preferably, no toxic or otherwise harmful degradation products are formed during preparation or storage of the formulations.

The solid dispersion comprising a cannabinoid, in particular cannabidiol, an amphiphilic block copolymer and a water-soluble film former leads to the formation of micelles upon contact with water or other aqueous media, such as gastrointestinal fluids. The micelles are essentially formed from the drug substance, surrounded by the solubilizing excipients.

One aspect is accordingly a micellar composition comprising an aqueous phase in which micelles are dispersed, which micelles comprise a cannabinoid, in particular cannabidiol, and solubilizing excipients, in particular the amphiphilic block copolymer and the water-soluble film former.

The amphiphilic block copolymer present in the formulations of the present invention acts as a solubilizer. The reference to an amphiphilic block copolymer includes the possibility that more than one such copolymer is present.

The cannabinoid and the amphiphilic block copolymer are present in the formulations comprising a cannabinoid, in particular cannabidiol, an amphiphilic block copolymer and a water-soluble film former in a weight ratio cannabinoid: amphiphilic block copolymer of typically 1 : 0.11 - 0.41, preferably 1 : 0.16 - 0.36, more preferably 1 : 0.21 - 0.31.

The amphiphilic block copolymers are solid at ambient temperature.

They have surfactant properties and, if used in appropriate concentration ranges in aqueous media, in particular water, can form micellar solutions.

In particular block copolymers containing at least one polyoxyethylene block and at least one polyoxypropylene block can be used.

Preferred block copolymers are poloxamers. Poloxamers are block copolymers whose molecular weights range from 1,100 to over 14,000. Different poloxamers differ only in the relative amounts of propylene and ethylene oxides added during manufacture.

In one embodiment, the solubilizer is Poloxamer 188 (Kolliphor P188; former brand name Lutrol F 68) / BASF; CAS No.: 9003-11-6).

Kolliphor P188 is a polyoxyethylene-polyoxypropylene block copolymer of the above general formula wherein n is approximately 79 and m is approximately 28.

Kolliphor P188 is available as a white to slightly yellowish waxy substance in the form of micropearls having a melting point of 52 - 57°C. It meets the requirements of Ph.Eur., USP / NF for Poloxamer 188.

As a further excipient, the formulations of the present invention contain a water-soluble film former. The reference to a water-soluble film former again includes the possibility that a combination of two or more such film formers is used.

Cannabidiol and the water soluble film former are present in a weight ratio cannabinoid : water soluble film former of typically 1 : 0.03 - 0.33, preferably 1 : 0.08 - 0.28, more preferably 1 : 0.13 - 0.23.

The water-soluble film former acts as a polymeric binder and additional solubilizer in the present formulation.

Examples of suitable water-soluble film formers are methyl cellulose (MC), hydroxypropyl methyl cellulose (HPMC), hydroxypropyl cellulose (HPC), hydroxyethyl cellulose (HEC), sodium carboxymethyl cellulose (Na-CMC) and polyvinyl pyrrolidone (PVP).

A preferred film former is PVP, in particular PVP K30 (such as Kollidon^{®} 30).

Another preferred film former is hydroxypropylmethyl cellulose (HPMC), in particular low-viscosity HPMC, such as HPMC with a viscosity of a 2% (w/w) aqueous solution at 20°C of 6 mPa·s or less.

The above discussed components are present in a weight ratio cannabidiol : amphiphilic block copolymer: water soluble film former (polyvinylpyrrolidone) of typically 1 : 0.11 - 0.41 : 0.03 - 0.33, preferably 1 : 0.16 - 0.36 : 0.08 - 0.28, more preferably 1 : 0.21 - 0.31 : 0.13 - 0.23.

It is in particular considered to further include an antioxidant or a combination of antioxidants to protect cannabidiol from oxidation.

Cannabinoids, in particular cannabidiol, are susceptible to oxidation. For instance, cannabidiol can be oxidized to monomeric and dimeric hydroxyquinones. The oxidation can lead to discoloration.

The oxidation can not only occur by molecular oxygen, but also by peroxides which may be introduced into the formulation by one or more of the excipients used.

Useful antioxidants which may be included into the formulation encompasses ascorbyl palmitate, alpha-tocopherol, butylhydroxytoluol (BHT, E321), butylhydroxyanisol (BHA, E320), ascorbic acid, and ethylenediaminetetraacetic acid (EDTA) sodium.

Ascorbyl palmitate is a preferred antioxidant. It can effectively suppress discoloration by oxidation.

The antioxidant is typically used in an amount of 0.5 to 2.5 wt%, preferably of 0.8 to 2 wt%, in particular 1.0 to 1.8 wt%, relative to the amount of the cannabinoid (in particular cannabidiol).

Other excipients may be present in addition.

In a preferred embodiment, the formulation contains in addition a diluent. Diluents (or fillers) as typically used in solid oral dosage forms can be employed. A preferred diluent is microcrystalline cellulose (such as Avicel^{®} PH 101). Another preferred diluent is mannitol (such as Pearlitol 160 C).

In formulations containing a diluent, there will typically be two phases, one phase comprising the active agent embedded in the polymeric excipients as detailed above and another phase comprising the diluent.

Active ingredient and diluent are typically present in a weight ratio cannabinoid (in particular cannabidiol) : diluent (in particular microcrystalline cellulose) of 1:0.5 - 2.7, preferably 1:0.9 - 2.3, in particular 1:1.3 - 1.9.

In a still further embodiments, silicon dioxide (such as Syloid^{®} 244 FP Silica) and/or colloidal silicon dioxide (such as Aerosil^{®} 200) are included in the formulation, in particular to serve as moisture adsorbents.

Active ingredient and total silicon dioxide components are typically present in a weight ratio cannabinoid (in particular cannabidiol) : total amount of all silicon dioxide components of 0.14 - 0.44, preferably 0.19 - 0.39, in particular 0.24 - 0.34.

While formulations according to the present invention are not limited to those containing the above discussed excipients, the formulations are preferably free or essentially free of triglycerides. Essentially free means that the formulation contains less than 5 % by weight, relative to all components, of triglycerides.

The solid dispersion is preferably free or essentially free of triglycerides. Essentially free means that the formulation contains less than 5 % by weight, relative to all components, of triglycerides.

Further, the solid dispersion is preferably free or essentially free of mono- and diglycerides. Essentially free means that the formulation contains less than 5 % by weight, relative to all components, of mono- and diglycerides.

Still further, the solid dispersion is preferably free or essentially free of fatty acids. Essentially free means that the formulation contains less than 5 % by weight, relative to all components, of fatty acids.

Preferably, the total amount of mono-, di- and triglycerides and fatty acids is less than 5 % by weight, relative to all components.

The present pharmaceutical formulations in the form of solid dispersions can be obtained by wet granulation techniques. The granulation can be carried out in a blender. Preferably, fluid bed granulation technology can be used.

According to the present invention, a method for preparing a cannabinoid containing formulation comprises the steps of (i) preparing a liquid composition comprising cannabidiol, the amphiphilic block copolymer and a solvent capable of at least partially dissolving the cannabidiol and the amphiphilic block copolymer; (ii) introducing the liquid composition into a fluid bed granulator; (iii) removing solvent to obtain a solid dispersion in particulate form; and (iv) recovering the solid dispersion in particulate form from the fluid bed granulator.

According to the invention, the liquid composition comprising cannabidiol, the amphiphilic block copolymer and the solvent preferably also comprises the water-soluble film former in at least partially dissolved form.

Further according to the invention, the liquid composition comprising cannabidiol the amphiphilic block copolymer and the solvent and optionally the water-soluble film former preferably also comprises the antioxidant in at least partially dissolved form.

The liquid composition may also comprise one or more further excipients. These can be present in any suitable form, for instance, in dissolved form or in dispersed form.

As an example, silicon dioxide can by present in the liquid composition in dispersed form. The cannabidiol and the excipients are preferably present in the liquid compositions in the weight ratios as indicated herein for the pharmaceutical formulations.

The solvent used to prepare the liquid composition can be any solvent capable of at least partially dissolving the cannabinoid, the amphiphilic block copolymer and preferably also the water-soluble film former and/or the antioxidant.

A preferred solvent is ethanol comprising not more than 10% v/v water, such as ethanol comprising not more than 4% v/v water, for instance, ethanol 96% v/v.

As indicated above, the liquid composition is introduced into a fluid bed granulator. In a preferred embodiment, the liquid composition is sprayed into a fluid bed granulator already containing solid particles.

The solid particles contained in the granulator can comprise one or more excipients. In a preferred embodiment, the solid particles comprise a diluent, such as microcrystalline cellulose.

One or more additional excipient, such as colloidal silicon dioxide, can also be present. The fluid bed granulator is operated so that solvent is removed and a solid dispersion in particulate form is obtained. For instance, an inlet air temperature of 45 ± 10°C can be chosen.

Solvent removal can be continued until a predetermined loss on drying (LOD) is reached. For instance, the product can be dried up to loss on drying of not more than 2.0%.

After drying the product is discharged and sieved.

The size of the granules obtained is not limited. Suitable sizes are in the range from 50 µm to 2000 µm, for example in the range from 100 µm to 1000 µm.

Formulations according to the present invention are preferably stable to discoloration. The color remains stable or changes only slightly to off-white upon storage for three months, preferably for six months and in particular for 12 months under long-term conditions (25°C/60% rh).

The granules represent a self-emulsifying solid dispersion. Upon combination with an aqueous medium a micellar solution can be obtained.

A formulation as described above, when subjected to an in vitro dissolution test in 0.1 N HCl + 2 % CTAB following the USP paddle method, releases at least 75 wt% of the cannabinoid within 60 minutes, preferably at least 90 wt% within 60 minutes. Further, the formulation releases at least 75 wt% of the cannabinoid within 45 minutes, preferably at least 85 wt% within 45 minutes.

The solid dispersion granules can be filled into bottles, sachets or stick packs using commercial standard technology and equipment. The solid dispersion granules are to be sprinkled on food or dispersed in a liquid, e.g., water.

A composition obtained by dispersing the solid dispersion granules in a liquid can be applied to patients being not able to swallow by means of a syringe through a gastric tube.

Depending on the final dosage strength per unit, the solid dispersion granules can also be filled into capsules which are feasible for swallowing (*e.g*. capsule size 2-1 for 25 mg/dose). Alternatively, for high dosed units, bigger capsules can be used as a primary packaging material for the granules. Such capsules are not for swallowing (*e.g*. capsule size up to 000 / sprinkle caps for 100-200 mg/dose). Rather, the solid dispersion granules are to be sprinkled on food or dispersed in a liquid, *e.g.*, water.

Alternatively, the solid dispersion granules can also be processed into tablets. The solid dispersion granules are combined with one or more excipients, such as a disintegrant, a glidant, and/or a lubricant. The obtained mixture is then compressed into tablets.

In one embodiment, they are processed into orally dispersible tablets.

### Examples

The invention is illustrated with the help of specific examples, without being restricted in any way thereby.

### Example 1

A cannabidiol containing granulate (solid dispersion) can be obtained using 20 parts by weight of cannabidiol and 80 parts by weight of Kolliphor P188. For preparing the granulate, the following options are available.

### Option (a)

The components are heated to a temperature of about 100°C. The melt is sprayed onto a solid sample of CBD in a fluidised bed at a product temperature of about 15 - 25°C. For this batch process, topspray, bottomspray and tangential spray configurations can be used.

### Option (b)

The components are heated to a temperature of about 100°C. The melt is sprayed into a fluidised bed apparatus which is initially empty. Solidification of the melt under fluidised bed conditions with a product temperature of about 15 - 25°C leads to the formation of a granulate. For this batch process, topspray, bottomspray and tangential spray configurations can be used.

### Option (c)

Preparation of a granulate from a melt can also be carried out continuously. This can be done by using the ProCell or MicroPx Technology (Glatt).

### Option (d)

The melt can also be processed in a spray tower. Using prilling nozzles, spherical particles of defined size can be obtained.

### Example 2

A cannabidiol containing granulate (solid dispersion) can be obtained using 30 parts by weight of cannabidiol and 70 parts by weight of Kolliphor P188. For preparing the granulate, the options outlined in Example 1 are available.

### Example 3

A cannabidiol containing granulate (solid dispersion) can be obtained using 40 parts by weight of cannabidiol and 60 parts by weight of Kolliphor P188. For preparing the granulate, the options outlined in Example 1 are available.

### Example 4

A cannabidiol containing granulate (solid dispersion) can be obtained using 20.05 parts by weight of cannabidiol, 76 parts by weight of Kolliphor P188, 3.4 parts by weight of Avicel PH 101, 0.5 parts by weight of Aerosil 200 and 0.05 parts by weight of BHT.

A melt from Kolliphor P188 and BHT having a temperature of about 100°C is sprayed onto a solid CBD, Avicel PH 101 and Aerosil 200 in a fluidised bed. The product temperature is about 15-25°C. For this batch process, topspray, bottomspray and tangential spray configurations can be used.

### Example 5

Compositions based on different weight ratios of CBD / solubilizer were prepared by melting and then cooling the melts. The compositions were analysed in terms of *in vitro* dissolution in 0.1N HCl following the USP paddle method.

For comparison the oily Cannabidiol solution according to DAC / NRF 22.10. and the commercial product Bionic Softgels were also tested.

CBD release after 60 min of in vitro dissolution testing in 0.1N HCl:

| | |
|---|---|
| CBD / Kolliphor P188 = 33/67; 200 mg CBD: | 69% drug release |
| CBD / Kolliphor P188 = 27/73; 200 mg CBD: | 82% drug release |
| CBD / Kolliphor P188 = 20/80; 200 mg CBD: | 96% drug release |
| CBD in oily (Miglyol 812) solution; 200 mg CBD: | 0% drug release |
| Bionic Softgels; 25 mg CBD | 96% drug release |

### Example 6

Tablets are prepared using 93.5 wt% of a granulate according to one of Examples 1 to 4, 5 wt% Polyplasone XL (disintegrant), 1 % Aerosil 200 (glidant) and 0.5 % magnesium stearate (lubricant).

### Example 7

### Preparation of granules

Cannabidiol (CBD) granules containing 29.7 % w/w active ingredient are prepared according to the following batch formula:

| Constituent | Function | Quantity (g) | Percentage in the final granulate (%) |
|---|---|---|---|
| CBD | drug substance | 170.78 | 29.7 |
| Poloxamer 188 *(Kolliphot*^{®} *P 188)* | solubilizer | 44.28 | 7.7 |
| Ascorbyl palmitate | antioxidant | 2.30 | 0.4 |
| Microcrystalline Cellulose *(Avicel*^{®} *PH 101)* | diluent | 278.30 | 48.4 |
| Colloidal silicon dioxide *(Aerosil*^{®} *200)* | moisture adsorbent | 46.00 | 8.0 |
| Polyvinylpyrrolidone *(Kollidon*^{®} *30)* | binder, solubilizer | 30.48 | 5.3 |
| Silicon Dioxide *(Syloid*^{®} *244 FP Silica)* | moisture adsorbent, glidant | 2.88 | 0.5 |
| Ethanol 96% v/v (a) | granulation liquid | 1150.00 | - |
| **Total theoretical weight** | | 575.02 | 100.0 |

In the first processing step, CBD and the pharmaceutical excipients poloxamer 188, ascorbyl palmitate, microcrystalline cellulose, silicon dioxide, colloidal silicon dioxide and polyvinylpyrrolidone are granulated.

For granulation, the fluid bed granulation technology is used.

The drug substance cannabidiol and the pharmaceutical excipients poloxamer 188, ascorbyl palmitate and polyvinylpyrrolidone are dissolved in ethanol 96% v/v. Silicon dioxide (Syloid^{®} 244 FP) is dispersed in the solution.

Microcrystalline cellulose and colloidal silicon dioxide (Aerosil^{®} 200) are charged into the fluid bed granulator and granulated with the described solution. The granules are discharged and sieved.

The volatile component ethanol 96% v/v is removed from the granules during the drying phase in the fluid bed dryer. The inlet air temperature is 45 ± 10°C, the product temperature 30 - 35°C.

The granules are dried up to a reference value for the loss in drying (LOD) percentage of not more than 2.0%.

### Dosage Form

Cannabidiol granules containing 29.7% w/w cannabidiol are filled in HDPE bottles to provide a total dose of 1500 mg Cannabidiol. The granulate is administered with 240 ml tap water (room temperature) in total. The granulate is firstly dispersed in 100 ml water. The remaining amount of water is used to rinse the container twice.

### Stability of the Cannabidiol Granulate

Samples are stored under accelerated conditions (40°C/75%), under intermediate conditions (30°C/65% rh) and under long-term conditions (25°C/60% rh).

Under storage at accelerated storage conditions the appearance discolored form white to yellowish after one months and to yellow after two months. The color changes only slightly to off-white at long-term conditions after three months and at intermediate conditions after four months.

The dissolution decreases slightly for storage at accelerated conditions after three months but is still well within specification. The dissolution remains unchanged after three months at long-term and after four months at intermediate conditions.

A decrease of assay of about 6 % at accelerated conditions after three months is observed, but the product is still within the shelf-life specification. At intermediate and long-term conditions no significant decrease of assay is observable after four months and three months, respectively.

As an impurity, an adduct of cannabidiol and ascorbyl palmitate is observed.

It is found to be at a level of 0.4 % at long-term and 0.5 % at accelerated conditions after three months of storage. At intermediate conditions the level is 0.5 % after four months.

A (Q)SAR assessment of the four possible structures of this adduct shows that its presence does not lead to an additional risk for patients if the formulation is administered using the doses and administrations schemes as disclosed herein.

### Stability of an Aqueous Dispersion

The chemical stability of an aqueous dispersion containing 1500 mg of cannabidiol was tested in a holding time study. For this purpose, about 5 g of a development batch (formulation without Aerosil 200) was dispersed in 240 ml water and stirred at ambient temperature. The impurity profile was monitored for 2 hours.

The impurity profile remains unchanged for the examined time period of two hours. Thus, the dispersion of the product in water for administration will be stable for a time period required for administration.

### CBD Release

Release is tested according to EP 2.9.3 / USP <711>. A paddle dissolution apparatus is used. Dissolution testing is performed at a standard temperature of 37°C ± 0,5°C and a stirrer speed of 100 rpm.

Complete release is observed in 0.1 M HCl + 2% (w/v) Cetyltrimethylammonium-bromide (CTAB) after 45 min.

### Example 8

Additional granulates were prepared following the method outlined in Example 7. Information on the composition is contained in the following table.

| | Batch | | | | | |
|---|---|---|---|---|---|---|
| | 200619 | 210076 | 210079 | 210112 | 210113 | 210114 |
| **Composition (wt %)** | | | | | | |
| Canapure PH | 29.7 | 29.7 | 29.7 | 29.7 | 30.4 | 31.6 |
| Avicel PH 101 | 48.4 | 48.4 | 49.7 | - | - | 46.0 |
| Pearlitol 160 C | - | - | - | 48.4 | 47.3 | - |
| Aerosil 200 | 8.0 | - | - | - | - | - |
| Syloid 244 FP | 0.5 | 8.5 | 12.5 | 8.5 | 8.5 | 8.1 |
| Kolliphor P 188 | 7.7 | 7.7 | 7.7 | 7.7 | 7.9 | 8.2 |
| PVP K-30 | 5.3 | 5.3 | - | 5.3 | 5.4 | 5.6 |
| Ascorbylpalmitat | 0.4 | 0.4 | 0.4 | 0.4 | 0.4 | 0.4 |
| | | | | | | |
| **Release after 45 min (wt %)** | 98.7 | 96.22 | 99.64 | 86.78 | 91.67 | 96.64 |

Pearlitol 160 C is a crystalline D mannitol powder having average mean particle diameter of 160 µm.

Release was determined using an in vitro dissolution method (1000 mL 0.1 M HCl + 2% (w/v) CTAB).

### Example 9

Pellets were made using the quantities of ingredients shown in Table 1 below.

For this purpose, 2-[1R-3-methyl-6R-(1-methylethenyl)-2-cyclohexen-1-yl]-5-pentyl-1,3-benzenediol (Canapure PH) was dissolved in ethanol 96%. This active ingredient has a log P of about 6.1.

Another solution was prepared by dissolving HPMC (Pharmacoat^{®} 603) in water.

The HPMC solution was then gradually added to the cannabidiol solution.

Then amorphous silicon dioxide (Syloid^{®} 244 FP) was added.

It was stirred with a propeller stirrer.

The spray liquid obtained was sprayed onto starter cores made of microcrystalline cellulose (Cellets^{®} 500).

This was done in a Mini-Glatt fluidized bed system with a Wurster insert. The inlet air temperature was 40°C. The average spray rate was 0.5 g/min.

**Table 1 - Substances and quantities used**

| **Formulation** | | **HPMC 0.8** | **HPMC 0.6** | **HPMC 0.3** |
|---|---|---|---|---|
| **Solids** | | Quantity | Quantity | Quantity |
| | Cellets 500 | 60.01 g / 81.5 % | 60.00 g / 72.7 % | 60.00 g / 72.7 % |
| | Canapure PH | 21.02 g / 16.1 % | 21.00 g / 24.2 % | 21.26 g / 24.5 % |
| | Pharmacoat 603 | 1.05 g / 0.8 % | 0.53 g / 0.6 % | 0.26 g / 0.3 % |
| | Syloid 244 FP | 2.10 g / 1.6 % | 2.10 g / 2.4 % | 2.10 g / 2.4 % |

| **Liquids (not included in the product)** | | | | |
|---|---|---|---|---|
| | Ethanol 96% | 79.81 g | 79.83 g | 79.82 g |
| | Pure water | 25.20 g | 25.21 g | 25.21 g |

| **Spray liquid** | | | | |
|---|---|---|---|---|
| | Solid content (wt./wt.) | 18.71 % | 18.36 % | 18.36 % |
| | Quantity sprayed | 72.80 g | 122.50 g | 122.50 g |

**Table 2 - Products**

| **Formulation** | **HPMC 0.8** | **HPMC 0.6** | **HPMC 0.3** |
|---|---|---|---|
| Theoretical yield | 73.63 g | 82.49 g | 82.49 g |
| Practical yield | 64.30 g / 87.33 % | 75.03 g / 90.95 % | 74.24 g / 90.00 % |
| Coating weight gain | 31.49 % | 66.82 % | 63.31 % |

### Example 10

The release from the pellet products obtained in Example 1 is examined using a blade stirrer apparatus in 1000 ml phosphate buffer pH 6.8 with an addition of 0.4% Tween^{®} 80, specifically at 37°C. The results obtained are shown in Fig. 2.

### Example 11

This example investigates the antiviral activity of Cannabidiol (CBD) against SARS-CoV-2 using a cell-culture based infection model. Two different cannabidiol compositions were tested (formulation of Example 7 and Canapure PH).

For each material stock solutions containing 10 mM cannabidiol in DMSO were prepared. The dissolved and filtered solutions were stored at room temperature for up to one week.

For the antiviral assay, Vero E6 cells and SARS-CoV-2 virus (isolate BetaCoV/Germany/BavPat1/2020 p.1) were incubated with different cannabidiol concentrations for 24 hours. Each experiment was run in triplicate.

More in particular, 25.000 Vero E6-cells per well were seeded into a 96-well plate. On the next day the CBD substances were diluted into culture medium (DMEM without FCS + 1 % Penicillin/Streptomycin) to obtain the desired final concentration (0 µM, 0.25 µM, 0.5 µM, 1 µM, 2.5 µM or 5 µM).

The medium was then removed from the cells and medium containing the different test concentrations was added to the cells.

In parallel about 150 FFU (focus forming units)/well were mixed with medium containing the different test concentrations.

After mixing the virus with the compounds, the cell culture medium from the 96-well plate with Vero E6-cells was removed.

200 µl sample from the virus containing plate were transferred to the Vero E6 plate.

The cells were incubated for 24 h at 37°C, 5 % CO2.

Residual viral activity was subsequently tested in cell culture by titration followed by staining of infected cells with a SARS-CoV-2 specific antibody. The positive cells were counted and the "focus forming units" (FFU) were calculated.

The results show that both substances (formulation of Example 7 and Canapure PH) were able to reduce the FFU values in a dose dependent manner, i.e., to inhibit the viral activity of SARS-CoV-2 in vitro. Differences between the substances were not statistically significant.

Detachment of cells at the highest concentration (5 µM) was observed for both substances. Detachment of cells was not visible in the respective control (DMSO), suggesting toxic effects of the substances at the indicated concentration. For the other concentrations tested, no toxic effects were observed.

To further characterise the inhibition of the viral activity, IC₅₀ values were calculated from the data determined as described above. The FFU values for the highest concentration (5 µM) were excluded, since the toxic effect on the cells may be interfering with the effectivity of the substances on the viral activity.

The tested substances showed inhibition of viral activity with IC₅₀ values of 1.015 µM (formulation of Example 7) and 0.789 µM (Canapure PH). The difference between these values was not statistically significant.

### Example 12 - Case Reports

A 49 year old male patient became infected with Covid-19 on November 26, 2020 and confirmed positive on November 30, 2020. The symptoms from the disease originally included low grade fever, chills, body aches and pains, lethargy and loss of appetite. The patient convalesced at home self-medicating with NSAIDs to control fever and pain, vitamin C and bed rest, drinking lots of fluids.

On December 4, 2020, the illness took a dramatic turn for the worse. The patient was unable to get a full breath and began becoming dizzy and hypoxic. The patient was admitted into a hospital and diagnosed with Covid Pneumonia.

Pulse oximetry showed an oxygen saturation of 86% oxygen. The inspiratory capacity was below 300mL. The patient was given IV fluids, a 5 days course of Remdesivir, heparin, dexamethasone and supplemental oxygen at 5L. The patient remained in the hospital until December 12, 2020, at which time he was discharged with supplemental home oxygen. The patient still had trouble breathing and could not maintain blood oxygen above 90% without the supplemental oxygen.

At that time the patient began taking 3g of the granulate of Example 7 mixed with orange juice on a daily basis. The patient noticed an immediate improvement in breathing, the tightness feeling in the chest was gone and in just 2 days the patient was able to maintain 96% oxygen levels without the supplemental oxygen. The patient's IC increased to 700mL and in a week he was able to reach 1500mL. The patient's other lingering symptoms, fatigue and lack of appetite were alleviated as well.

A 20 year old male patient became infected with Covid-19. He took the granulate of Example 7 for three days. During this time, he experienced only mild COVID symptoms. After stopping CBD intake for one day, symptoms worsened. Taking CBD again from day 5 onwards reduced the symptoms again.

## Claims

1. A cannabinoid for use in the treatment of a patient suffering from an infection with SARS-CoV-2 or of a subject at risk to be infected with SARS-CoV-2, wherein the cannabinoid is cannabidiol (2-[(1R,6R)-3-methyl-6-(1-methylethenyl)-2-cyclohexen-1-yl]-5-pentyl-1,3-benzenediol).

2. A cannabinoid for use in the treatment according to claim 1, wherein the treatment is for preventing or ameliorating the cytokine release syndrome (CRS) and/or for reducing the viral load; and/or wherein the treatment is for preventing or ameliorating the cytokine release syndrome (CRS); and/or wherein the treatment reduces the serum IL-6 level; and/or wherein the treatment is for preventing or ameliorating the acute respiratory distress syndrome (ARDS).

3. A cannabinoid for use in the treatment according to any of the proceeding claims, wherein the treatment is initiated during the non-severe symptomatic period.

4. A cannabinoid for use in the treatment according to any of the proceeding claims, wherein the treatment is initiated if the patient is diagnosed with at least one symptom of disease selected from fever, dry cough, shortness of breath, and evidence of rales/crackles on physical examination, myalgia, fatigue, dyspnea, anorexia, loss of sense of smell and taste, and nephritis.

5. A cannabinoid for use in the treatment according to any of claims 1 to 3, wherein the treatment is initiated if a patient shows pathological lung features either by CT-scan or chest x-ray;
or wherein the treatment is initiated if the patient is diagnosed with at least one symptom of disease selected from fever, dry cough, shortness of breath, and evidence of rales/crackles on physical examination, myalgia, fatigue, dyspnea, anorexia, loss of sense of smell and taste, and nephritis; and shows pathological lung features either by CT-scan or chest x-ray;
or wherein the treatment is initiated based on a reduced saturation of peripheral oxygen (SpO2), in particular wherein the treatment is initiated if the patient shows a saturation of peripheral oxygen (SpO2) of ≤93% at rest in ambient air or requires between 3L/min and 5L/min of oxygen to maintain SpO2 >97%;
or wherein the treatment is initiated upon worsening of lung involvement, defined as worsening of oxygen saturation >3 percentage points or decrease in PaO2 (partial pressure of oxygen, arterial) >10%, with stable FiO2 (fraction of inspired oxygen) in the last 24h;
or wherein the treatment is initiated based on one or more of serum IL-6 ≥ 5.4 pg/ml; CRP level >70 mg/L (without other confirmed infectious or non-infectious course); CRP level >= 40 mg/L and doubled within 48 hours (without other confirmed infectious or non-infectious course); lactate dehydrogenase > 250 U/L; D-dimer > 1 µg/mL; serum ferritin > 300 µg/mL;
or wherein the treatment is initiated if the patient is diagnosed with at least one symptom of disease selected from fever, dry cough, shortness of breath, and evidence of rales/crackles on physical examination, myalgia, fatigue, dyspnea, anorexia, loss of sense of smell and taste, and nephritis; and shows at least one laboratory finding selected from serum IL-6 ≥ 5.4 pg/ml; CRP level >70 mg/L (without other confirmed infectious or non-infectious course); CRP level >= 40 mg/L and doubled within 48 hours (without other confirmed infectious or non-infectious course); lactate dehydrogenase > 250 U/L; D-dimer > 1 µg/mL; serum ferritin > 300 µg/mL;
or wherein the treatment is initiated if the patient shows thrombocytopenia < 120.000 x 10E9/L, and/or a lymphocyte count < 0.6 x 10E9/L;
or wherein the treatment is initiated if the patient is diagnosed with at least one symptom of disease selected from fever, dry cough, shortness of breath, and evidence of rales/crackles on physical examination, myalgia, fatigue, dyspnea, anorexia, loss of sense of smell and taste, and nephritis; and/or shows at least one laboratory finding selected from serum IL-6 ≥ 5.4 pg/ml; CRP level >70 mg/L (without other confirmed infectious or non-infectious course); CRP level >= 40 mg/L and doubled within 48 hours (without other confirmed infectious or non-infectious course); lactate dehydrogenase > 250 U/L; D-dimer > 1 µg/mL; serum ferritin > 300 µg/mL; and shows thrombocytopenia < 120.000 x 10E9/L, and/or a lymphocyte count < 0.6 x 10E9/L.

6. A cannabinoid for use in the treatment according to any of the proceeding claims, wherein the patient belongs to a risk group, wherein the patient suffers from adipositas.

7. A cannabinoid for use in the treatment according to any of the proceeding claims, wherein the cannabinoid is applied in combination with one or more antiviral agents selected from remdesivir (an inhibitor of the RNA polymerase of the virus) and ritonavir/lopinavir (an HIV medicament); in combination with a drug against idiopathic pulmonary fibrosis; or in combination with a drug against blood clots or a drug against cardiac arrhythmias.

8. A cannabinoid for use in the treatment according to any of the proceeding claims, wherein the cannabinoid is administered orally.

9. A cannabinoid for use in the treatment according to any of the proceeding claims, wherein the cannabinoid is administered at a dose between 150 mg and 5000 mg one to four times per day, such as between 250 mg and 5000 mg one to four times per day, in particular wherein the dose is 375 mg, 750 mg, 1500 mg, or 3000 mg, and this dose is administered one to four times per day; and more in particular wherein the dose is 375 mg, 750 mg, 1500 mg, or 3000 mg, and this dose is administered BID.

10. A cannabinoid for use in the treatment according to any of the proceeding claims, wherein the cannabinoid is administered BID at a dose of 1500 mg.

11. A cannabinoid for use in the treatment according to any of the proceeding claims, wherein the cannabinoid is formulated as a solid dispersion.

12. A cannabinoid for use in the treatment according to claim 11, wherein the solid dispersion comprises the cannabinoid and a solubilizer which is an amphiphilic block copolymer capable of forming a micellar solution if combined with an aqueous medium.

13. A cannabinoid for use in the treatment according to any of claims 11 and 12, wherein the solubilizer is a block copolymer containing at least one polyoxyethylene block and at least one polyoxypropylene block, in particular wherein the solubilizer is a poloxamer, in particular poloxamer 188.

14. A cannabinoid for use in the treatment according to any of claims 12 and 13, wherein the cannabinoid and the solubilizer are present in a weight ratio cannabinoid: solubilizer of 1:0.2 - 10.0, preferably 1:0.5 - 6.0, in particular 1:1 - 5.

15. A cannabinoid for use in the treatment according to any of claims 11 to 14, wherein the solid dispersion in addition comprises an antioxidant, in particular wherein the antioxidant is used in an amount of 0.5 to 2.5 wt%, preferably of 0.8 to 2 wt%, in particular 1.0 to 1.8 wt%, relative to the amount of the cannabinoid and wherein the antioxidant is preferably ascorbyl palmitate.

16. A cannabinoid for use in the treatment according to claim 11, wherein the solid dispersion comprises in admixture a cannabinoid, an amphiphilic block copolymer as a solubilizer and a water-soluble film former, in particular wherein the cannabinoid and the amphiphilic block copolymer are present in a weight ratio cannabinoid: amphiphilic block copolymer of 1 : 0.11 - 0.41, preferably 1 : 0.16 - 0.36, more preferably 1 : 0.21 - 0.31; and/or
wherein the amphiphilic block copolymer is a block copolymer containing at least one polyoxyethylene block and at least one polyoxypropylene block, in particular wherein the amphiphilic block copolymer is a poloxamer, in particular poloxamer 188; and/or
wherein the cannabinoid and the water soluble film former are present in a weight ratio cannabinoid : water soluble film former of 1 : 0.03 - 0.33, preferably 1 : 0.08 - 0.28, more preferably 1 : 0.13 - 0.23; and/or
wherein the water-soluble film former is polyvinylpyrrolidone; and/or wherein the water soluble film former is hydroxypropylmethyl cellulose; and/or
wherein the components are present in a weight ratio cannabinoid: amphiphilic block copolymer : water soluble film former of 1 : 0.11 - 0.41 : 0.03 - 0.33, preferably 1 : 0.16 - 0.36 : 0.08 - 0.28, more preferably 1 : 0.21 - 0.31 : 0.13 - 0.23; and/or
wherein the solid dispersion in addition comprises an antioxidant, in particular wherein the antioxidant is used in an amount of 0.5 to 2.5 wt%, preferably of 0.8 to 2 wt%, in particular 1.0 to 1.8 wt%, relative to the amount of the cannabinoid and wherein the antioxidant is optionally ascorbyl palmitate; and/or
wherein the solid dispersion comprises a diluent, in particular wherein the cannabinoid and the diluent are present in a weight ratio cannabinoid: diluent of 1:0.5 - 2.7, preferably 1:0.9 - 2.3, in particular 1:1.3 - 1.9 and wherein the diluent is optionally microcrystalline cellulose and/or mannitol; and/or wherein the solid dispersion comprises a moisture adsorbent, in particular wherein the cannabinoid and the moisture adsorbent are present in a weight ratio cannabinoid : moisture adsorbent of 0.14 - 0.44, preferably 0.19 - 0.39, in particular 0.24 - 0.34 and the moisture adsorbent optionally comprises a silicon dioxide; and/or
wherein the solid dispersion is free or essentially free of triglycerides; and/or mono- and diglycerides; and/or fatty acids.

17. A cannabinoid for use in the treatment according to claim 16, wherein the formulation, when subjected to an in vitro dissolution test in 0.1N HCl + 2 % CTAB following the USP paddle method, releases at least 75 wt% of the cannabinoid within 60 minutes, in preferably at least 90 wt% within 60 minutes; and/or
wherein the formulation, when subjected to an in vitro dissolution test in 0.1N HCl + 2% CTAB following the USP paddle method, releases at least 75 wt% of the cannabinoid within 45 minutes, preferably at least 85 wt% within 45 minutes.

18. A cannabinoid for use in the treatment according to any of claims 1 to 10, wherein the cannabinoid is incorporated in a formulation comprising a core and a coating on the core, wherein the coating comprises the cannabinoid, one or more water-soluble film formers and not more than 20 wt.-%, based on the weight of all components, of other excipients, in particular wherein hydroxypropylmethyl cellulose (HPMC) is used as the water-soluble film former; and/or
wherein the film former/film formers, based on the total amount of cannabinoid, is/are comprised in a total proportion of 0.3-10 wt.-%; and/or
wherein more than 30 wt.-% and less than 80 wt.-% of the cannabinoid contained is released within two hours; and /or wherein more than 40 wt.-% and less than 90 wt.-% of the cannabinoid contained is released within three hours; and/or wherein more than 50 wt.-% and less than 95 wt.-% of the cannabinoid contained is released within four hours.

## Patentansprüche

1. Ein Cannabinoid zur Verwendung bei der Behandlung eines Patienten, der an einer Infektion mit SARS-CoV-2 leidet, oder einer Person, bei der das Risiko einer Infektion mit SARS-CoV-2 besteht, wobei es sich bei dem Cannabinoid um Cannabidiol (2-[(1R,6R)-3-Methyl-6-(1-methylethenyl)-2-cyclohexen-1-yl]-5-pentyl-1,3-benzendiol) handelt.

2. Ein Cannabinoid zur Verwendung bei der Behandlung gemäß Anspruch 1, wobei die Behandlung der Vorbeugung oder Linderung des Zytokinfreisetzungssyndroms (CRS) und/oder der Verringerung der Viruslast dient; und/oder wobei die Behandlung der Vorbeugung oder Linderung des Zytokinfreisetzungssyndroms (CRS) dient; und/oder
wobei die Behandlung den IL-6-Spiegel im Serum senkt; und/oder
wobei die Behandlung der Vorbeugung oder Linderung des akuten Atemnotsyndroms (ARDS) dient.

3. Ein Cannabinoid zur Verwendung bei der Behandlung gemäß einem der vorstehenden Ansprüche, wobei die Behandlung während der Phase mit nicht schweren Symptomen eingeleitet wird.

4. Ein Cannabinoid zur Verwendung bei der Behandlung gemäß einem der vorstehenden Ansprüche, wobei die Behandlung eingeleitet wird, wenn bei dem Patienten mindestens ein Krankheitssymptom diagnostiziert wird, das aus Fieber, trockenem Husten, Atemnot und Anzeichen von Rasselgeräuschen bei der körperlichen Untersuchung, Myalgie, Müdigkeit, Dyspnoe, Anorexie, Verlust des Geruchs- und Geschmackssinns sowie Nephritis ausgewählt ist.

5. Ein Cannabinoid zur Verwendung bei der Behandlung gemäß einem der Ansprüche 1 bis 3, wobei die Behandlung eingeleitet wird, wenn bei einem Patienten pathologische Lungenmerkmale entweder mittels CT-Scan oder Röntgenaufnahme des Brustkorbs festgestellt werden;
oder wobei die Behandlung eingeleitet wird, wenn bei dem Patienten mindestens ein Krankheitssymptom diagnostiziert wird, ausgewählt aus Fieber, trockenem Husten, Atemnot und Anzeichen von Rasselgeräuschen bei der körperlichen Untersuchung, Myalgie, Müdigkeit, Dyspnoe, Anorexie, Verlust des Geruchs- und Geschmackssinns sowie Nephritis; und pathologische Lungenmerkmale entweder mittels CT-Scan oder Röntgenaufnahme des Brustkorbs aufweist;
oder wobei die Behandlung auf der Grundlage einer verminderten peripheren Sauerstoffsättigung (SpO2) eingeleitet wird, insbesondere wobei die Behandlung eingeleitet wird, wenn der Patient in Ruhe an der Umgebungsluft eine periphere Sauerstoffsättigung (SpO2) von ≤ 93 % aufweist oder zwischen 3 l/min und 5 l/min Sauerstoff benötigt, um einen SpO2-Wert von > 97 % aufrechtzuerhalten;
oder wobei die Behandlung bei einer Verschlechterung der Lungenbeteiligung eingeleitet wird, definiert als eine Verschlechterung der Sauerstoffsättigung um >3 Prozentpunkte oder eine Abnahme des PaO2 (arterieller Sauerstoffpartialdruck) um >10 % bei stabilem FiO2 (Fraktion des eingeatmeten Sauerstoffs) in den letzten 24 Stunden;
oder wobei die Behandlung auf der Grundlage eines oder mehrerer der folgenden Kriterien eingeleitet wird: Serum-IL-6 ≥ 5,4 pg/ml; eines CRP-Wertes > 70 mg/L (ohne anderen bestätigten infektiösen oder nicht-infektiösen Verlauf); eines CRP-Wertes ≥ 40 mg/L, der sich innerhalb von 48 Stunden verdoppelt hat (ohne anderen bestätigten infektiösen oder nicht-infektiösen Verlauf); eines Laktatdehydrogenase-Wertes > 250 U/L; D-Dimer > 1 µg/ml; Serumferritin > 300 µg/ml;
oder wobei die Behandlung eingeleitet wird, wenn bei dem Patienten mindestens ein Krankheitssymptom diagnostiziert wird, ausgewählt aus Fieber, trockenem Husten, Atemnot und Anzeichen von Rasselgeräuschen bei der körperlichen Untersuchung, Myalgie, Müdigkeit, Dyspnoe, Anorexie, Verlust des Geruchs- und Geschmackssinns sowie Nephritis; und mindestens einen Laborbefund aufweist, ausgewählt aus: Serum-IL-6 ≥ 5,4 pg/ml; CRP-Wert > 70 mg/l (ohne anderen bestätigten infektiösen oder nicht-infektiösen Verlauf); CRP-Wert ≥ 40 mg/l, der sich innerhalb von 48 Stunden verdoppelt hat (ohne anderen bestätigten infektiösen oder nicht-infektiösen Verlauf); Laktatdehydrogenase > 250 U/l; D-Dimer > 1 µg/ml; Serumferritin > 300 µg/ml;
oder wobei die Behandlung eingeleitet wird, wenn der Patient eine Thrombozytopenie < 120.000 × 10⁹/l und/oder eine Lymphozytenzahl < 0,6 × 10⁹/l aufweist;
oder wobei die Behandlung eingeleitet wird, wenn bei dem Patienten mindestens ein Krankheitssymptom diagnostiziert wird, das aus Fieber, trockenem Husten, Atemnot und Anzeichen von Rasselgeräuschen bei der körperlichen Untersuchung, Myalgie, Müdigkeit, Dyspnoe, Anorexie, Verlust des Geruchs- und Geschmackssinns sowie Nephritis ausgewählt ist; und/oder wenn mindestens ein Laborbefund vorliegt, der aus einem Serum-IL-6-Spiegel ≥ 5,4 pg/ml; CRP-Wert > 70 mg/l (ohne anderen bestätigten infektiösen oder nicht-infektiösen Verlauf); CRP-Wert ≥ 40 mg/l, der sich innerhalb von 48 Stunden verdoppelt hat (ohne anderen bestätigten infektiösen oder nicht-infektiösen Verlauf); Laktatdehydrogenase > 250 U/l; D-Dimer > 1 µg/ml; Serumferritin > 300 µg/ml; sowie eine Thrombozytopenie < 120.000 × 10⁹/l und/oder eine Lymphozytenzahl < 0,6 × 10⁹/l aufweist.

6. Ein Cannabinoid zur Verwendung bei der Behandlung gemäß einem der vorstehenden Ansprüche, wobei der Patient einer Risikogruppe angehört und an Adipositas leidet.

7. Ein Cannabinoid zur Verwendung bei der Behandlung gemäß einem der vorstehenden Ansprüche, wobei das Cannabinoid in Kombination mit einem oder mehreren antiviralen Wirkstoffen, ausgewählt aus Remdesivir (einem Inhibitor der RNA-Polymerase des Virus) und Ritonavir/Lopinavir (einem HIV-Medikament), in Kombination mit einem Arzneimittel gegen idiopathische Lungenfibrose oder in Kombination mit einem Arzneimittel gegen Blutgerinnsel oder einem Arzneimittel gegen Herzrhythmusstörungen angewendet wird.

8. Ein Cannabinoid zur Verwendung bei der Behandlung gemäß einem der vorstehenden Ansprüche, wobei das Cannabinoid oral verabreicht wird.

9. Ein Cannabinoid zur Verwendung bei der Behandlung gemäß einem der vorstehenden Ansprüche, wobei das Cannabinoid in einer Dosis zwischen 150 mg und 5000 mg ein- bis viermal täglich verabreicht wird, beispielsweise zwischen 250 mg und 5000 mg ein- bis viermal täglich, insbesondere wobei die Dosis 375 mg, 750 mg, 1500 mg oder 3000 mg beträgt und diese Dosis ein- bis viermal täglich verabreicht wird; und insbesondere, wobei die Dosis 375 mg, 750 mg, 1500 mg oder 3000 mg beträgt und diese Dosis zweimal täglich (BID) verabreicht wird.

10. Ein Cannabinoid zur Verwendung bei der Behandlung gemäß einem der vorstehenden Ansprüche, wobei das Cannabinoid zweimal täglich (BID) in einer Dosis von 1500 mg verabreicht wird.

11. Ein Cannabinoid zur Verwendung bei der Behandlung gemäß einem der vorstehenden Ansprüche, wobei das Cannabinoid als feste Dispersion formuliert ist.

12. Ein Cannabinoid zur Verwendung bei der Behandlung gemäß Anspruch 11, wobei die feste Dispersion das Cannabinoid und einen Lösungsvermittler umfasst, bei dem es sich um ein amphiphiles Blockcopolymer handelt, das in der Lage ist, in Verbindung mit einem wässrigen Medium eine mizellare Lösung zu bilden.

13. Ein Cannabinoid zur Verwendung bei der Behandlung gemäß einem der Ansprüche 11 und 12, wobei der Lösungsvermittler ein Blockcopolymer ist, das mindestens einen Polyoxyethylenblock und mindestens einen Polyoxypropylenblock enthält, insbesondere wobei der Lösungsvermittler ein Poloxamer ist, insbesondere Poloxamer 188.

14. Ein Cannabinoid zur Verwendung bei der Behandlung gemäß einem der Ansprüche 12 und 13, wobei das Cannabinoid und der Lösungsvermittler in einem Gewichtsverhältnis Cannabinoid:Lösungsvermittler von 1:0,2 bis 10,0, vorzugsweise 1:0,5 bis 6,0, insbesondere 1:1 bis 5, vorliegen.

15. Ein Cannabinoid zur Verwendung bei der Behandlung gemäß einem der Ansprüche 11 bis 14, wobei die feste Dispersion zusätzlich ein Antioxidans umfasst, insbesondere wobei das Antioxidans in einer Menge von 0,5 bis 2,5 Gew.-%, vorzugsweise von 0,8 bis 2 Gew.-%, insbesondere von 1,0 bis 1,8 Gew.-%, bezogen auf die Menge des Cannabinoids verwendet wird, und wobei das Antioxidans vorzugsweise Ascorbylpalmitat ist.

16. Ein Cannabinoid zur Verwendung bei der Behandlung gemäß Anspruch 11, wobei die feste Dispersion eine Mischung aus einem Cannabinoid, einem amphiphilen Blockcopolymer als Lösungsvermittler und einem wasserlöslichen Filmbildner umfasst, insbesondere wobei das Cannabinoid und das amphiphile Blockcopolymer in einem Gewichtsverhältnis Cannabinoid: amphiphiles Blockcopolymer von 1 : 0,11 - 0,41, vorzugsweise 1 : 0,16 - 0,36, noch bevorzugter 1 : 0,21 - 0,31 vorliegen; und/oder wobei das amphiphile Blockcopolymer ein Blockcopolymer ist, das mindestens einen Polyoxyethylenblock und mindestens einen Polyoxypropylenblock enthält, insbesondere wobei das amphiphile Blockcopolymer ein Poloxamer ist, insbesondere Poloxamer 188;
und/oder wobei das Cannabinoid und der wasserlösliche Filmbildner in einem Gewichtsverhältnis von Cannabinoid : wasserlöslicher Filmbildner von 1 : 0,03 bis 0,33, vorzugsweise 1 : 0,08 bis 0,28, noch bevorzugter 1 : 0,13 bis 0,23 vorliegen;
und/oder wobei der wasserlösliche Filmbildner Polyvinylpyrrolidon ist; und/oder wobei der wasserlösliche Filmbildner Hydroxypropylmethylcellulose ist;
und/oder wobei die Komponenten in einem Gewichtsverhältnis von Cannabinoid : amphiphiles Blockcopolymer : wasserlöslicher Filmbildner von 1 : 0,11 - 0,41 : 0,03-0,33, vorzugsweise 1 : 0,16 - 0,36 : 0,08 - 0,28, noch bevorzugter 1 : 0,21 - 0,31 : 0,13 - 0,23 vorliegen;
und/oder wobei die feste Dispersion zusätzlich ein Antioxidans umfasst, insbesondere wobei das Antioxidans in einer Menge von 0,5 bis 2,5 Gew.-%, vorzugsweise von 0,8 bis 2 Gew.-%, insbesondere von 1,0 bis 1,8 Gew.-%, bezogen auf die Menge des Cannabinoids, eingesetzt wird und wobei das Antioxidans gegebenenfalls Ascorbylpalmitat ist;
und/oder wobei die feste Dispersion ein Verdünnungsmittel umfasst, insbesondere wobei das Cannabinoid und das Verdünnungsmittel in einem Gewichtsverhältnis Cannabinoid : Verdünnungsmittel von 1 : 0,5 - 2,7, vorzugsweise 1 : 0,9 - 2,3, insbesondere 1 : 1,3 - 1,9 vorliegen und wobei das Verdünnungsmittel gegebenenfalls mikrokristalline Cellulose und/oder Mannitol ist;
und/oder wobei die feste Dispersion ein Feuchtigkeitsadsorbens umfasst, insbesondere wobei das Cannabinoid und das Feuchtigkeitsadsorbens in einem Gewichtsverhältnis Cannabinoid : Feuchtigkeitsadsorbens von 0,14 bis 0,44, vorzugsweise von 0,19 bis 0,39, insbesondere von 0,24 bis 0,34 vorliegen und das Feuchtigkeitsadsorbens gegebenenfalls Siliciumdioxid umfasst;
und/oder wobei die feste Dispersion frei oder im Wesentlichen frei von Triglyceriden ist; und/oder von Mono- und Diglyceriden; und/oder von Fettsäuren ist.

17. Ein Cannabinoid zur Verwendung bei der Behandlung gemäß Anspruch 16, wobei die Formulierung, wenn sie einem in-vitro-Auflösungstest in 0,1 N HCl + 2 % CTAB nach der USP-Paddelmethode unterzogen wird, innerhalb von 60 Minuten mindestens 75 Gew.-% des Cannabinoids freisetzt, vorzugsweise mindestens 90 Gew.-% innerhalb von 60 Minuten;
und/oder wobei die Formulierung, wenn sie einem in-vitro-Auflösungstest in 0,1 N HCl + 2 % CTAB nach der USP-Paddelmethode unterzogen wird, innerhalb von 45 Minuten mindestens 75 Gew.-% des Cannabinoids freisetzt, vorzugsweise mindestens 85 Gew.-% innerhalb von 45 Minuten.

18. Ein Cannabinoid zur Verwendung bei der Behandlung gemäß einem der Ansprüche 1 bis 10, wobei das Cannabinoid in einer Formulierung enthalten ist, die einen Kern und eine Beschichtung auf dem Kern umfasst, wobei die Beschichtung das Cannabinoid, einen oder mehrere wasserlösliche Filmbildner und nicht mehr als 20 Gew.-%, bezogen auf das Gewicht aller Bestandteile, an anderen Hilfsstoffen umfasst, wobei insbesondere Hydroxypropylmethylcellulose (HPMC) als wasserlöslicher Filmbildner verwendet wird;
und/oder wobei der Filmbildner bzw. die Filmbildner, bezogen auf die Gesamtmenge an Cannabinoid, in einem Gesamtanteil von 0,3-10 Gew.-% umfasst ist bzw. sind;
und/oder wobei mehr als 30 Gew.-% und weniger als 80 Gew.-% des enthaltenen Cannabinoids innerhalb von zwei Stunden freigesetzt werden; und/oder wobei mehr als 40 Gew.-% und weniger als 90 Gew.-% des enthaltenen Cannabinoids innerhalb von drei Stunden freigesetzt werden; und/oder wobei mehr als 50 Gew.-% und weniger als 95 Gew.-% des enthaltenen Cannabinoids innerhalb von vier Stunden freigesetzt werden.

## Revendications

1. Cannabinoïde pour utilisation dans le traitement d'un patient souffrant d'une infection par le SARS-CoV-2 ou d'un sujet présentant un risque d'infection par le SARS-CoV-2, ledit cannabinoïde étant le cannabidiol (2-[(1R,6R)-3-méthyl-6-(1-méthyléthényl)-2-cyclohexén-1-yl]-5-pentyl-1,3-benzènediol).

2. Cannabinoïde pour utilisation dans le traitement selon la revendication 1, dans lequel le traitement vise à prévenir ou à atténuer le syndrome de libération de cytokines (CRS) et/ou à réduire la charge virale; et/ou
dans lequel le traitement vise à prévenir ou à atténuer le syndrome de libération de cytokines (CRS); et/ou
dans lequel le traitement réduit le taux sérique d'IL-6; et/ou dans lequel le traitement vise à prévenir ou à atténuer le syndrome de détresse respiratoire aiguë (SDRA).

3. Cannabinoïde pour utilisation dans le traitement selon l'une quelconque des revendications précédentes, dans lequel le traitement est initié pendant la période symptomatique non grave.

4. Cannabinoïde pour utilisation dans le traitement selon l'une quelconque des revendications précédentes, dans lequel le traitement est initié si le patient présente au moins un symptôme de la maladie choisi parmi la fièvre, la toux sèche, l'essoufflement, la présence de râles ou de crépitements à l'examen physique, la myalgie, la fatigue, la dyspnée, l'anorexie, la perte de l'odorat et du goût, et la néphrite.

5. Cannabinoïde pour utilisation dans le traitement selon l'une quelconque des revendications 1 à 3, dans lequel le traitement est initié si un patient présente des signes pathologiques pulmonaires mis en évidence soit par tomodensitométrie, soit par radiographie thoracique;
ou dans lequel le traitement est initié si le patient présente au moins un symptôme de la maladie choisi parmi la fièvre, la toux sèche, l'essoufflement, la présence de râles ou de crépitements à l'examen physique, les myalgies, la fatigue, la dyspnée, l'anorexie, la perte de l'odorat et du goût, et la néphrite; et présente des signes pathologiques pulmonaires mis en évidence soit par tomodensitométrie, soit par radiographie thoracique;
ou dans lequel le traitement est initié sur la base d'une saturation en oxygène périphérique (SpO2) réduite, en particulier lorsque le traitement est instauré si le patient présente une saturation en oxygène périphérique (SpO2) ≤ 93 % au repos à l'air ambiant ou nécessite entre 3 L/min et 5 L/min d'oxygène pour maintenir une SpO2 > 97 %;
ou dans lequel le traitement est initié en cas d'aggravation de l'atteinte pulmonaire, définie comme une aggravation de la saturation en oxygène > 3 points de pourcentage ou une diminution de la PaO2 (pression partielle d'oxygène artérielle) > 10 %, avec une FiO2 (fraction d'oxygène inspirée) stable au cours des dernières 24 heures;
ou dans lequel le traitement est initié sur la base d'un ou plusieurs des critères suivants : un taux sérique d'IL-6 ≥ 5,4 pg/ml; un taux de CRP > 70 mg/L (en l'absence d'autre évolution infectieuse ou non infectieuse confirmée); un taux de CRP ≥ 40 mg/L ayant doublé en 48 heures (en l'absence d'autre évolution infectieuse ou non infectieuse confirmée); une lactate déshydrogénase > 250 U/L; un taux de D-dimères > 1 µg/mL; un taux de ferritine sérique > 300 µg/mL;
ou dans lequel le traitement est initié si le patient présente au moins un symptôme de la maladie choisi parmi la fièvre, la toux sèche, l'essoufflement et la présence de râles ou de crépitements à l'examen physique, les myalgies, la fatigue, la dyspnée, l'anorexie, la perte de l'odorat et du goût, et la néphrite; et présente au moins un résultat de laboratoire choisi parmi : un taux sérique d'IL-6 ≥ 5,4 pg/ml; un taux de CRP > 70 mg/L (en l'absence d'autre évolution infectieuse ou non infectieuse confirmée); un taux de CRP ≥ 40 mg/L ayant doublé en 48 heures (en l'absence d'autre évolution infectieuse ou non infectieuse confirmée); une lactate déshydrogénase > 250 U/L; un taux de D-dimères > 1 µg/mL; un taux de ferritine sérique > 300 µg/mL;
ou dans lequel le traitement est initié si le patient présente une thrombocytopénie < 120 000 × 10E9/L et/ou un nombre de lymphocytes < 0,6 × 10E9/L;
ou dans lequel le traitement est initié si le patient présente au moins un symptôme de la maladie choisi parmi la fièvre, la toux sèche, l'essoufflement et la présence de râles/craquements à l'examen physique, la myalgie, la fatigue, la dyspnée, l'anorexie, la perte de l'odorat et du goût, et la néphrite; et/ou présente au moins un résultat de laboratoire choisi parmi un taux sérique d'IL-6 ≥ 5,4 pg/ml; un taux de CRP > 70 mg/L (en l'absence d'autre évolution infectieuse ou non infectieuse confirmée); un taux de CRP ≥ 40 mg/L ayant doublé en 48 heures (en l'absence d'autre évolution infectieuse ou non infectieuse confirmée); une lactate déshydrogénase > 250 U/L; un taux de D-dimères > 1 µg/mL; un taux de ferritine sérique > 300 µg/mL; et présente une thrombocytopénie < 120 000 × 10⁹/L, et/ou un nombre de lymphocytes < 0,6 × 10⁹/L.

6. Cannabinoïde pour utilisation dans le traitement selon l'une quelconque des revendications précédentes, dans lequel le patient appartient à un groupe à risque, et dans lequel le patient souffre d'adipositas.

7. Cannabinoïde pour utilisation dans le traitement selon l'une quelconque des revendications précédentes, dans lequel le cannabinoïde est administré en association avec un ou plusieurs agents antiviraux choisis parmi le remdesivir (un inhibiteur de la polymérase ARN du virus) et le ritonavir/lopinavir (un médicament contre le VIH); en association avec un médicament contre la fibrose pulmonaire idiopathique; ou en association avec un médicament contre les caillots sanguins ou un médicament contre les arythmies cardiaques.

8. Cannabinoïde pour utilisation dans le traitement selon l'une quelconque des revendications précédentes, dans lequel le cannabinoïde est administré par voie orale.

9. Cannabinoïde pour utilisation dans le traitement selon l'une quelconque des revendications précédentes, dans lequel le cannabinoïde est administré à une dose comprise entre 150 mg et 5000 mg, une à quatre fois par jour, par exemple entre 250 mg et 5000 mg, une à quatre fois par jour, en particulier lorsque la dose est de 375 mg, 750 mg, 1500 mg ou 3000 mg, et cette dose est administrée une à quatre fois par jour; et plus particulièrement dans lequel la dose est de 375 mg, 750 mg, 1500 mg ou 3000 mg, et cette dose est administrée deux fois par jour (BID).

10. Cannabinoïde pour utilisation dans le traitement selon l'une quelconque des revendications précédentes, dans lequel le cannabinoïde est administré deux fois par jour à une dose de 1500 mg.

11. Cannabinoïde pour utilisation dans le traitement selon l'une quelconque des revendications précédentes, dans lequel le cannabinoïde est formulé sous forme de dispersion solide.

12. Cannabinoïde pour utilisation dans le traitement selon la revendication 11, dans lequel la dispersion solide comprend le cannabinoïde et un solubilisant qui est un copolymère à blocs amphiphile capable de former une solution micellaire lorsqu'il est combiné avec un milieu aqueux.

13. Cannabinoïde pour utilisation dans le traitement selon l'une quelconque des revendications 11 et 12, dans lequel l'agent solubilisant est un copolymère à blocs contenant au moins un bloc polyoxyéthylène et au moins un bloc polyoxypropylène, en particulier dans lequel l'agent solubilisant est un poloxamère, en particulier le poloxamère 188.

14. Cannabinoïde pour utilisation dans le traitement selon l'une quelconque des revendications 12 et 13, dans lequel le cannabinoïde et l'agent solubilisant sont présents dans un rapport pondéral cannabinoïde : agent solubilisant compris entre 1 : 0,2 et 10,0, de préférence entre 1 : 0,5 et 6,0, en particulier entre 1 : 1 et 5.

15. Cannabinoïde pour utilisation dans le traitement selon l'une quelconque des revendications 11 à 14, dans lequel la dispersion solide comprend en outre un antioxydant, en particulier dans lequel l'antioxydant est utilisé en une quantité de 0,5 à 2,5 % en poids, de préférence de 0,8 à 2 % en poids, en particulier de 1,0 à 1,8 % en poids, par rapport à la quantité du cannabinoïde et dans laquelle l'antioxydant est de préférence le palmitate d'ascorbyle.

16. Cannabinoïde pour utilisation dans le traitement selon la revendication 11, dans lequel la dispersion solide comprend, en mélange, un cannabinoïde, un copolymère à blocs amphiphile servant de solubilisant et un agent filmogène hydrosoluble, en particulier dans lequel le cannabinoïde et le copolymère à blocs amphiphile sont présents dans un rapport pondéral cannabinoïde : copolymère à blocs amphiphile de 1 : 0,11 à 0,41, de préférence de 1 : 0,16 à 0,36, et de manière encore plus préférentielle de 1 : 0,21 à 0,31;
et/ou dans laquelle le copolymère à blocs amphiphile est un copolymère à blocs contenant au moins un bloc polyoxyéthylène et au moins un bloc polyoxypropylène, en particulier lorsque le copolymère à blocs amphiphile est un poloxamère, en particulier le poloxamère 188; et/ou
dans lequel le cannabinoïde et l'agent filmogène hydrosoluble sont présents dans un rapport pondéral cannabinoïde : agent filmogène hydrosoluble compris entre 1 : 0,03 et 0,33, de préférence entre 1 : 0,08 et 0,28, et de manière encore plus préférentielle entre 1 : 0,13 et 0,23; et/ou
dans lequel l'agent filmogène hydrosoluble est la polyvinylpyrrolidone; et/ou dans lequel l'agent filmogène hydrosoluble est l'hydroxypropylméthylcellulose; et/ou
dans lequel les composants sont présents dans un rapport pondéral cannabinoïde : copolymère séquencé amphiphile : agent filmogène hydrosoluble de 1 : 0,11 à 0,41 : 0,03 à 0,33, de préférence de 1 : 0,16 à 0,36 : 0,08 à 0,28, plus préférablement de 1 : 0,21 à 0,31 : 0,13 à 0,23; et/ou
dans laquelle la dispersion solide comprend en outre un antioxydant, en particulier dans laquelle l'antioxydant est utilisé en une quantité de 0,5 à 2,5 % en poids, de préférence de 0,8 à 2 % en poids, en particulier de 1,0 à 1,8 % en poids, par rapport à la quantité du cannabinoïde, et dans laquelle l'antioxydant est éventuellement du palmitate d'ascorbyle; et/ou
dans laquelle la dispersion solide comprend un diluant, en particulier dans laquelle le cannabinoïde et le diluant sont présents dans un rapport pondéral cannabinoïde : diluant compris entre 1 : 0,5 et 2,7, de préférence entre 1 : 0,9 et 2,3, en particulier entre 1 : 1,3 et 1,9, et dans laquelle le diluant est éventuellement de la cellulose microcristalline et/ou du mannitol; et/ou
dans laquelle la dispersion solide comprend un adsorbant d'humidité, en particulier dans laquelle le cannabinoïde et l'adsorbant d'humidité sont présents dans un rapport pondéral cannabinoïde : adsorbant d'humidité compris entre 0,14 et 0,44, de préférence entre 0,19 et 0,39, en particulier entre 0,24 et 0,34, et l'adsorbant d'humidité comprend éventuellement du dioxyde de silicium; et/ou
dans laquelle la dispersion solide est exempte ou pratiquement exempte de triglycérides; et/ou de mono- et diglycérides; et/ou d'acides gras.

17. Cannabinoïde pour utilisation dans le traitement selon la revendication 16, dans lequel la formulation, lorsqu'elle est soumise à un essai de dissolution in vitro dans du HCl 0,1 N + 2 % de CTAB selon la méthode à palette de l'USP, libère au moins 75 % en poids du cannabinoïde en 60 minutes, de préférence au moins 90 % en poids en 60 minutes; et/ou
dans laquelle la formulation, lorsqu'elle est soumise à un essai de dissolution in vitro dans du HCl 0,1 N + 2 % de CTAB selon la méthode de l'USP à la palette, libère au moins 75 % en poids du cannabinoïde en 45 minutes, de préférence au moins 85 % en poids en 45 minutes.

18. Cannabinoïde pour utilisation dans le traitement selon l'une quelconque des revendications 1 à 10, dans lequel le cannabinoïde est incorporé dans une formulation comprenant un noyau et un enrobage sur le noyau, dans laquelle l'enrobage comprend le cannabinoïde, un ou plusieurs agents filmogènes hydrosolubles et pas plus de 20 % en poids, par rapport au poids de tous les composants, d'autres excipients, en particulier dans laquelle de l'hydroxypropylméthylcellulose (HPMC) est utilisée comme agent filmogène hydrosoluble; et/ou
dans laquelle le ou les agents filmogènes, par rapport à la quantité totale de cannabinoïde, sont présents dans une proportion totale de 0,3 à 10 % en poids; et/ou
dans laquelle plus de 30 % en poids et moins de 80 % en poids du cannabinoïde contenu sont libérés en l'espace de deux heures; et/ou dans laquelle plus de 40 % en poids et moins de 90 % en poids du cannabinoïde contenu sont libérés en l'espace de trois heures; et/ou dans laquelle plus de 50 % en poids et moins de 95 % en poids du cannabinoïde contenu sont libérés en l'espace de quatre heures.
